# EUROPEAN PATENT SPECIFICATION

(11) **EP 2 323 995 B1**
(45) Date of publication and mention of the grant of the patent: **21.11.2012**
(21) Application number: 09781761.3
(22) Date of filing: 12.08.2009
(51) Int. Cl.: C07D 265/16, C07D 413/06

(54) **POLYMERIZABLE BENZOXAZINE COMPOSITIONS**
POLYMERISIERBARE BENZOXAZINZUSAMMENSETZUNGEN
COMPOSITIONS POLYMÉRISABLES DE BENZOXAZINE

(30) Priority: 14.08.2008 EP 08014512
(43) Date of publication of application: 25.05.2011
(73) Proprietor: Henkel AG & Co. KGaA, 40589 Düsseldorf (DE)
(72) Inventor: SUDO, Atsushi, Tokyo 177-0044 (JP); KUDOH, Ryoichi, Shiga 520-0002 (JP); ENDO, Takeshi, Yokohama 220-0006 (JP); TADEN, Andreas, 40597 Düsseldorf (DE)
(86) International application number: PCT/EP2009/060448
(87) International publication number: WO 2010/018198

(56) References cited:
- WO-A-01/34581
- WO-A-03/072638
- WO-A-2005/100432
- WO-A-2007/064801
- WO-A-2008/060545
- JP-A- 2007 169 587
- JP-A- 2008 050 557
- JP-A- 2008 195 907
- US-A1- 2007 184 323
- US-B1- 6 482 946
- B. KISKAN ET AL.: "Synthesis, characterization, and properties of new thermally curable polyetheresters containing benzoxazine moieties in the main chain" JOURNAL OF POLYMER SCIENCE, PART A: POLYMER CHEMISTRY, vol. 46, no. 2, 2007, pages 414-420, XP002554139
- A. NAGAI ET AL.: "Synthesis and crosslinking behavior of a novel linear polymer bearing 1,2,3-triazol and benzoxazine groups in the main chain by a step-growth click-coupling reaction" JOURNAL OF POLYMER SCIENCE, PART A: POLYMER CHEMISTRY, vol. 46, no. 7, 2008, pages 2316-2325, XP002554140

## Description

### FIELD OF THE INVENTION

The present invention relates to polymerizable compositions, comprising at least two specific benzoxazines and to cured products obtained from the polymerizable compositions. More particularly, the present invention relates to increasing the polymerization rate of polymerizable compositions via incorporation of at least one specific benzoxazine.

### DESCRIPTION OF THE PRIOR ART

Benzoxazines have been reported in literature as generally having a high glass transition temperature, good electrical properties (e.g. dielectric constant), and low flammability.

Normally, benzoxazines are cured at relatively high temperatures. In order to reduce the polymerization temperature of benzoxazines various catalysts, like phenols (JP2000-178332A), carboxylic acids (JP2001-213957A, US 6,376,080 B1), amines (JP2000-86863A,), imidazoles (JP 2000-178332A), and phosphines (JP 2003-82099A) have been reported. US 6,225,440 B1 discloses Lewis acids, such as PCl₅, TiCl₄, AlCl₃ as highly active catalysts for the polymerization of benzoxazines.

However, in practical applications, such strong acids negatively contribute to the final polymerization result and its practical properties. For example deterioration of chemical resistance and physical properties of the cured material may appear. Additionally, Lewis acids, such as PCl₅, TiCl₄, AlCl₃ are highly sensitive to moisture and could cause the formation of volatile, toxic and corrosive impurities.

Notwithstanding the state of the technology, it would be desirable to create benzoxazine-based polymerizable compositions, which can be cured in an environmentally friendly process at relatively low temperatures in short time periods, without using toxic and/or corrosive compounds.

### SUMMARY OF THE INVENTION

The inventors of the present invention surprisingly found, that a polymerizable composition, comprising two different benzoxazines can be cured in an environmentally friendly process at relatively low temperatures in short time periods, without using any additional catalyst.

Therefore, the present invention provides a polymerizable composition, comprising
a) at least one benzoxazine compound A according to formula (I), 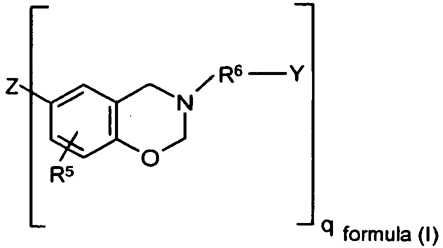 wherein q is an integer from 1 to 4, Z is selected from the group consisting of a direct bond (when q is 2), hydrogen (when q is 1), alkyl (when q is 1), alkylene (when q is 2 to 4), carbonyl (when q is 2), oxygen (when q is 2), thiol (when q is 1), sulfur (when q is 2), sulfoxide (when q is 2), and sulfone (when q is 2), Y is selected from the group consisting of a hydroxyl group, and a nitrogen-containing heterocycle, R⁶ is a linear or branched divalent alkylene group, comprising 1 to 15 carbon atoms, which may be interrupted by one or more heteroatom(s), selected from oxygen, nitrogen and sulfur and R⁵ is selected from hydrogen, halogen, alkyl, alkenyl or R⁵ is a divalent residue creating a naphthoxazine residue out of the benzoxazine structure, and
b) at least one benzoxazine compound B, which is different from benzoxazine compound A. This means that it does not correspond to formula (I), but has a different molecular structure.

The polymerizable compositions are in particular suitable as coatings, adhesives, sealants and matrices for the preparation of reinforced material such as prepregs and towpregs and/or can be used in injection molding or extrusion.

Therefore it is another object of the invention to provide an adhesive, sealant or coating, comprising the polymerizable composition of the present invention and a cured reaction product of the polymerizable composition of the present invention, in particular a cured reaction product containing bundles or layers of fibers. It is further provided a method of preparing such material.

In another object of the present invention the at least one benzoxazine compound according to formula (I) is used as curatives for curable compositions. It is further provided a method to increase the polymerization rate of a curable composition at temperatures up to 250°C

### DETAILED DESCRIPTION OF THE INVENTION

The polymerizable composition of the present invention comprises at least two different benzoxazine compounds A and B.

The at least one benzoxazine compound A is represented by formula (I), 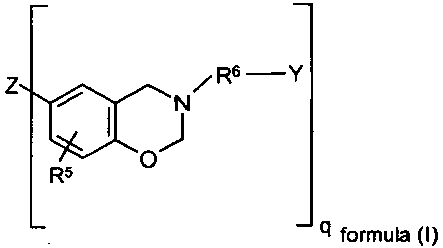 wherein q is an integer from 1 to 4, Z is selected from the group consisting of a direct bond (when q is 2), hydrogen (when q is 1), alkyl (when q is 1), alkylene (when q is 2 to 4), carbonyl (when q is 2), oxygen (when q is 2), thiol (when q is 1), sulfur (when q is 2), sulfoxide (when q is 2), and sulfone (when q is 2), Y is selected from the group consisting of a hydroxyl group, and a nitrogen-containing heterocycle, R⁶ is a linear or branched divalent alkylene group, comprising 1 to 15 carbon atoms, which may be interrupted by one or more heteroatom(s), selected from oxygen, nitrogen and sulfur and R⁵ is selected from hydrogen, halogen, alkyl, alkenyl or R⁵ is a divalent residue creating a naphthoxazine residue out of the benzoxazine structure.

The term "interrupted by", as used in the present invention, refers to a divalent alkylene group R⁶ in which a carbon atom of the divalent alkylene group is replaced by a heteroatom selected from the group consisting of *-S-* (sulfur),*-O-* (oxygen), and *-NH-* (nitrogen), whereby said carbon atom is not a terminal atom of said alkylene group.

In certain embodiment of the present invention R⁵ is selected from the group consisting of hydrogen, methyl, ethyl, n-propyl, iso-propyl, n-butyl, sec-butyl and iso-butyl. In a particular preferred embodiment R⁵ is selected from hydrogen or methyl.

The divalent alkylene group R⁶ includes saturated or unsaturated, substituted or unsubstituted divalent alkylene groups.
In a preferred embodiment of the present invention the alkylene group R⁶ is not interrupted by one or more heteroatom(s), selected from oxygen, nitrogen and sulfur, and contains from 2 to about 8 carbon atoms. Preferably R⁶ can be selected from linear divalent alkylene groups, such as ethylene, propylene, butylene, pentylene, and hexylene.

In certain embodiments of the present invention R⁶ is a linear divalent alkylene group, comprising 1 to 4 carbon atoms, preferably 2 to 3 carbon atoms.

In another preferred embodiment of the present invention, the divalent alkylene group R⁶ is interrupted by oxygen. Particular preferably R⁶ is a linear divalent alkylene group interrupted by at least one oxygen atom. More preferably R⁶ is a linear divalent alkylene group interrupted by one oxygen heteroatom and said alkylene group comprises 3 to 6 carbon atoms.

if the divalent alkylene group R⁶ is interrupted by more than one heteroatom, such as oxygen, it is preferred that the shortest atom chain between two heteroatoms contains at least 2 consecutive carbon atoms.

Benzoxazine compounds A according to formula (I), wherein the divalent alkylene group R⁶ is interrupted by one or more heteroatom(s), such as oxygen are advantageous, because said benzoxazine compounds show an improved water-solubility and could therefore be used for water-based applications.

If Y is a hydroxyl group, the at least one benzoxazine compound A according to formula (I) carries a hydroxyl-functionalized residue. Therefore, these kinds of benzoxazine compounds are named as hydroxyl-functionalized benzoxazine compounds in the present invention.

Specific examples for hydroxyl-functionalized benzoxazine compounds include:

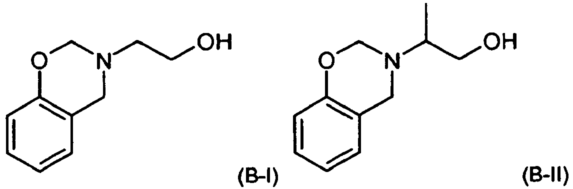

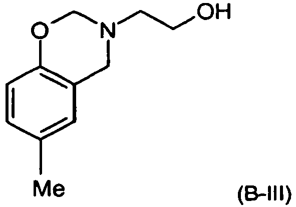

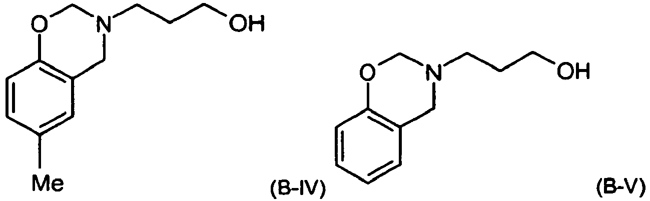

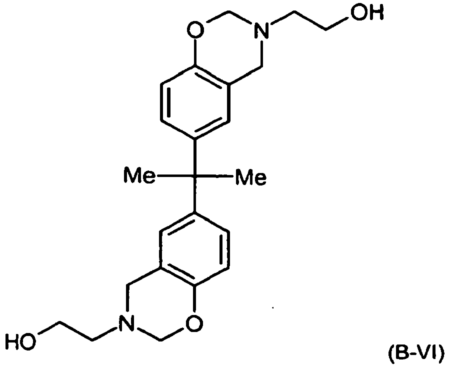

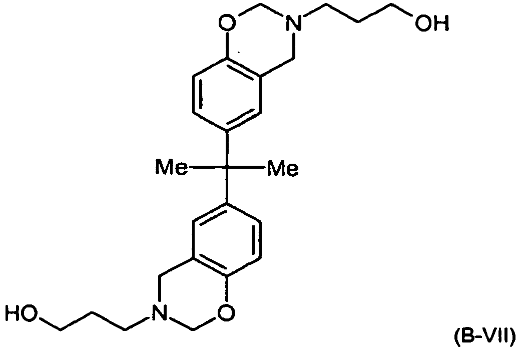

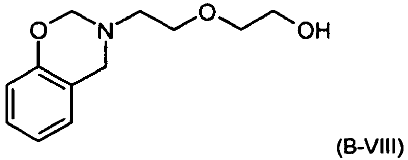

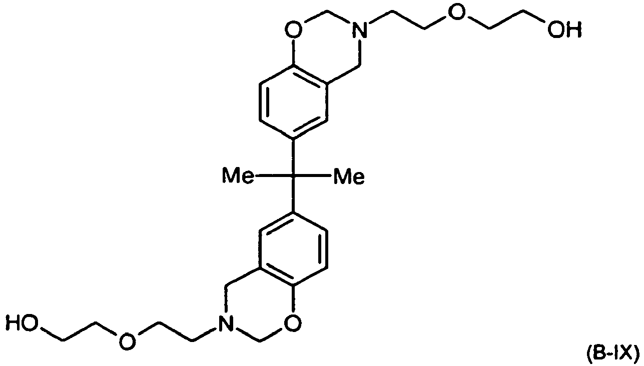

Hydroxyl-functionalized benzoxazine compounds according to formula (I) can typically be prepared according to any method as e.g. the method disclosed in the Japanese patent application JP 2002-302486 on page 11, line 66-100. Said method relies on the reaction of a phenolic compound, with an aldehyde, such as formaldehyde and aliphatic amino alcohol. The reaction time can vary from a few minutes to a few hours, depending on reactant concentration, reactivity and temperature. Alternatively, a method for preparing the hydroxyl-functionalized benzoxazines according to formula (II) is disclosed by Kiskan and Yagci in Polymer 46 (2005), pp 11690 - 11697 and by Kiskan, Yagci and Ishida in Journal of Polymer Science: Part A: Polymer Chemistry (2008), vol. 46, pp 414- 420.

Examples of suitable phenolic compounds include substituted or unsubstituted mono- or bisphenolic compounds, such as phenol, 4-methyl-phenol, bisphenol A, bisphenol F, bisphenol S and thiodiphenol.

Examples of suitable aliphatic amino alcohols include 2-aminoethyl alcohol, 3-amino-1-propanol, amino-2-propanol, 4-amino-1-butanol, 2-amino-1-butanol, 4-amino-2-butanol, 5-amino-1-pentanol. 6-amino-1-hexanol, 7-amino-1-heptanol, 3-amino-1,2-propanediol, and 2-(2-aminoethoxy)ethanol all commercially available from Sigma-Aldrich and 2-amino-1,3-propanediol, commercially available from Tokyo Chemical Industry.

Despite the above described synthesis, alternative synthesis depending on the nature of the starting reactants can be used to prepare the hydroxyl-functionalized benzoxazine compounds according to formula (I).

As a preferred alternative, the at least one benzoxazine compound A according to formula (I) carries a nitrogen-containing heterocycle as residue Y.

The term "nitrogen-containing heterocycle" as used in the present invention relates to monocyclic ring structures having 3 to 8 ring atoms, preferably 5 to 6 ring atoms and comprising at least one nitrogen heteroatom and at least two carbon atoms. Said nitrogen-containing heterocycle includes saturated or unsaturated, substituted or unsubstituted ring systems and may additionally comprise other hetero atom(s) such as sulfur and oxygen. The nitrogen-containing heterocycle is connected to the benzoxazine moiety via residue R⁶, whereas R⁶ can be attached to any carbon or any nitrogen ring atom by formally replacing a hydrogen atom that is covalently bound to a carbon or nitrogen ring atom.

Preferred nitrogen-containing heterocycles of the present invention may be selected from 5-membered heterocycles, such as imidazoles, imidazolidones, tetrazoles, oxazoles pyrroles, pyrrolines, pyrrolidines, and pyrazoles, or 6-membered heterocycles, such as piperidines, piperidones, piperazines, pyridines, diazines and morpholines.

Particularly preferably, the nitrogen-containing heterocycle is an aromatic and/or unsubstituted heterocycle, preferably comprising 5 or 6 rings atoms. In particular the nitrogen-containing heterocycle preferably comprises at least two nitrogen heteroatoms.

If Y is a nitrogen-containing heterocycle, the at least one benzoxazine compound A according to formula (I) carries a heterocyclic residue. Therefore, these kinds of benzoxazine compounds are named as heterocyclic-functionalized benzoxazine compounds in the present invention.

The most preferred heterocyclic residue Y in formula (I) is a substituted or unsubstituted imidazole and said imidazole is connected to the benzoxazine moiety via residue R⁶, whereas R⁶ can be attached to any carbon atom or one nitrogen-atom of said imidazole.

Said imidazole-functionalized benzoxazine compounds according to formula (I) are particular preferred components of the polymerizable composition of the present invention.

Therefore specific examples for heterocyclic-functionalized benzoxazine compounds are represented by formula (III): 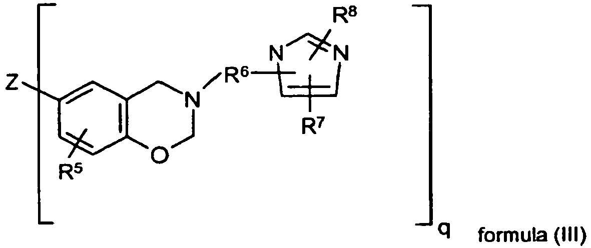 wherein q, Z, R⁶ and R⁵ are defined as above and R⁷ and R⁸ are independently selected from hydrogen, alkyl, alkenyl, and aryl.

In a certain embodiment of the present invention R⁷ and R⁸ are independently selected from the group consisting of hydrogen, methyl, ethyl, n-propyl, iso-propyl, n-butyl, sec-butyl and iso-butyl. In a particular preferred embodiment of the present invention R⁷ and R⁸ are independently selected from the group consisting of hydrogen and methyl.

Representative heterocyclic-functionalized benzoxazine compounds within formula (III) include the following structures: 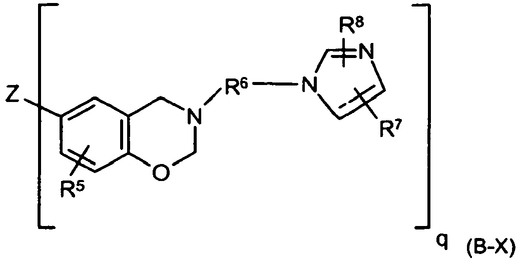 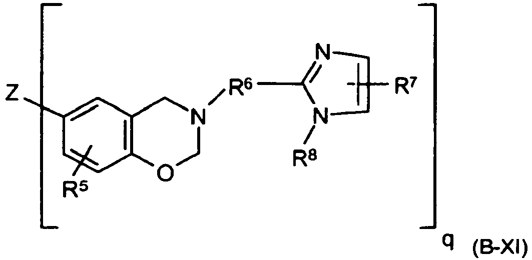 where q, Z, R⁵, R⁶, R⁷ and R⁸ are defined as above.

In a particular preferred embodiment of the present invention R⁶ is attached to one nitrogen-atom of the imidazole heterocycle as shown in structure (B-X).

Specific examples of the above generically described heterocyclic-functionalized benzoxazine compounds according to formula (III) include:

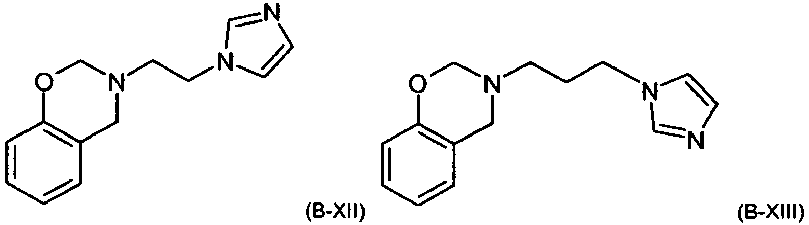

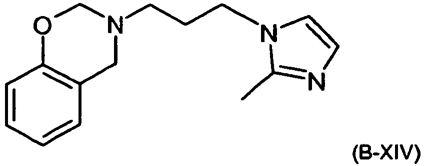

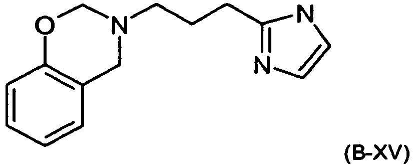

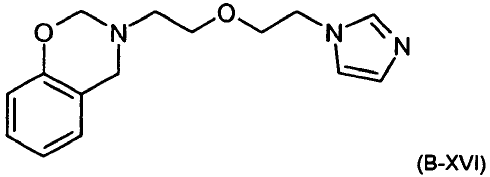

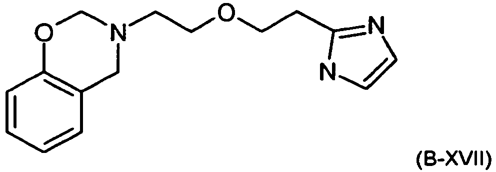

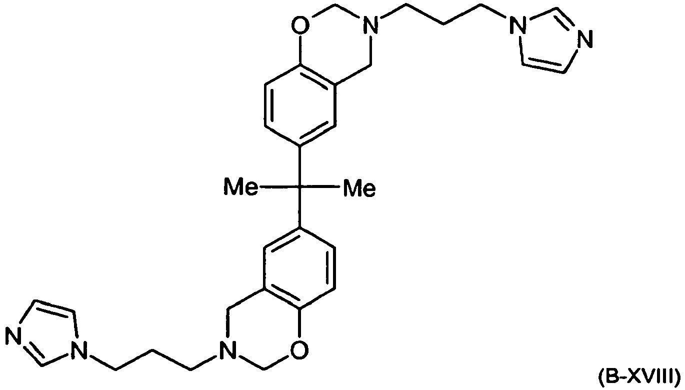

The above-shown heterocyclic-functionalized benzoxazine compounds according to formula (III) may typically be prepared by reacting at least one phenolic compound, with an aldehyde, such as formaldehyde and at least one aminoalkylimidazole compound.

Examples of suitable phenolic compounds include substituted or unsubstituted mono- or bisphenolic compounds, such as phenol, 4-methyl-phenol, bisphenol A, bisphenol F, bisphenol S and thiodiphenol.

For the present invention it is particularly desirable to use formaldehyde as an aldehyde.

Although all the formaldehyde may be provided as formalin, this is an undesirable method because formalin is expensive and it introduces an unnecessary amount of water into the system which must be removed later. However employing formalin in addition to paraformaldehyde in preparing the benzoxazine according to formula (II) is advantageous. Paraformaldehyde is preferred as it is significantly less expensive than formalin. The solid paraformaldehyde, however, is insoluble in nearly all organic solvents. This forces the reaction to occur at the solid-liquid interface, thus significantly limiting the rate of reaction. Employing formalin in combination with the paraformaldehyde provides enough water and methanol to dissolve the paraformaldehyde. Alternatively, just water may be used. Thus it is advantageous to employ a paraformaldehyde/formalin ratio of at least 1:1, based on the dry weight of the formaldehyde, and preferably of about 8:1 and more. However taking into account the abovementioned drawback of slow reaction, formaldehyde can be employed in water-free form such as paraformalehyde, trioxane or polyoxymethylene only, paraformaldehyde being most preferred.

Representative aminoalkylimidazole compounds of the present invention include compounds of general structure (IV), 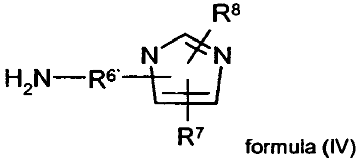 wherein R^{6'} is a linear or branched divalent alkylene group, comprising 1 to 15 carbon atoms, which may be interrupted by one or more heteroatom(s), selected from oxygen, nitrogen and sulfur and R⁷ and R⁸ are defined as above.

The term "interrupted by" as used in the present invention refers to a divalent alkylene group R^{6'} in which a carbon atom of the divalent alkylene group is replaced by a heteroatom selected from the group consisting of *-S-* (sulfur),*-O-* (oxygen), and *-NH-* (nitrogen), whereby said carbon atom is not a terminal atom of said alkylene group.

The divalent alkylene group R^{6'} includes saturated or unsaturated, substituted or unsubstituted divalent alkylene groups.

In a preferred embodiment of the present invention the alkylene group R^{6'} is not interrupted by one or more heteroatom(s), selected from oxygen, nitrogen and sulfur, and contains from 2 to about 8 carbon atoms. Preferably R^{6'} can be selected from linear divalent alkylene groups, such as ethylene, propylene, butylene, pentylene, and hexylene.

In certain embodiments of the present invention R^{6'} is a linear divalent alkylene group, comprising 1 to 4 carbon atoms, preferably 2 to 3 carbon atoms.

In another preferred embodiment of the present invention, the divalent alkylene group R^{6'} is interrupted by oxygen. Particular preferably R^{6'} is a linear divalent alkylene group interrupted by at least one oxygen atom. More preferably R^{6'} is a linear divalent alkylene group interrupted by one oxygen heteroatom and said alkylene group comprises 3 to 6 carbon atoms.
If the divalent alkylene group R^{6'} is interrupted by more than one heteroatom, such as oxygen, it is preferred that the shortest atom chain between two heteroatoms contains at least 2 consecutive carbon atoms.

Representative aminoalkylimidazole compounds within formula (IV) include the following structures: 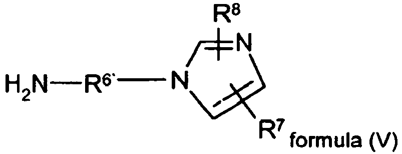 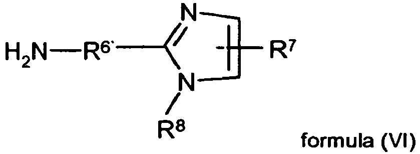 where R^{6'} R⁷, and R⁸ are as defined above.

In a particular preferred embodiment of the present invention R^{6'} is attached to one nitrogen-atom of the imidazole heterocycle as shown in formula (V).

The 1-aminoalkylimidazole compounds of the formula (V) are likewise generally known compounds which are available as commercial products.
Examples for commercially available 1-aminoalkylimidazole compounds are 1-(3-aminopropyl)imidazole, available commercially under the tradename Lupragen® API form the BASF SE, 3-imidazol-1-yl-2-methyl-propylamine, available commercially form ChemPacific, 2-methyl-1H-imidazole-1-propanamine, available commercially form 3B Scientific Corporation, 3-imidazol-1-yl-2-hydroxy-propylamine, available commercially form Ambinter, ParisCollection, 1-(4-aminobutyl)imidazole, available commercially form Ambinter, Paris, 2-ethyl-1H-imidazole-1-propanamine, available commercially form ChemBridge Corp..

If desired, however, instead of using commercially available sources, the 1-aminoalkylimidazole compounds can be prepared in accordance with the process described in Houben-Weyl, Methoden der organischen Chemie [Methods of Organic Chemistry] vol. E 16d, p. 755 et seq., Georg-Thiemeverlag Stuttgart, 1992.

The 2-aminoalkylimidazoles of formula (VI) are likewise generally known compounds. They can be prepared, for example, in accordance with the synthesis procedure described in Tetrahedron 2005, vol 61, on pp 11148-11155.

In some preferred embodiments of the present invention the polymerizable composition of the present invention may comprise mixtures of different hydroxyl-functionalized benzoxazine compounds A or mixtures of different heterocyclic-functionalized benzoxazine compounds A. Additionally, in certain embodiments mixtures of at least one hydroxyl-functionalized benzoxazine and at least one heterocyclic-functionalized benzoxazine can be used in the present invention.

In a preferred embodiment of the present invention benzoxazine compound B is different from benzoaxazine compounds of formula (I).

In a particular preferred embodiment of the present invention the at least one benzoxazine compound B, which is different from benzoxazine compound A, can comprise at least one benzoxazine according to formula (II) and/or formula (IIa) 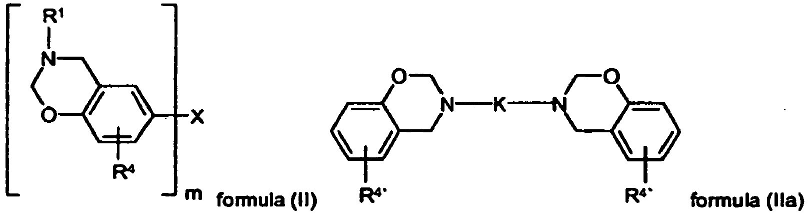 wherein m is an integer from 1 to 4, X is selected from the group consisting of a direct bond (when m is 2), hydrogen (when m is 1), alkyl (when m is 1), alkylene (when m is 2 to 4), carbonyl (when m is 2), oxygen (when m is 2), thiol (when m Is 1), sulfur (when m is 2), sulfoxide (when m is 2), and sulfone (when m is 2), R¹ is selected from hydrogen, alkyl, alkenyl and aryl and R¹ does not comprise any hydroxyl group or any nitrogen-containing heterocycle, K is selected from the group consisting of biphenyl, diphenyl methane, diphenyl isopropane, diphenyl sulfide, diphenyl sulfoxide, diphenyl sulfone, and diphenyl ketone, and R⁴ is selected from hydrogen, halogen, alkyl, alkenyl or R⁴ is a divalent residue creating a naphthoxazine residue out of the benzoxazine structure and R^{4'} is defined as R⁴

As defined above the term "nitrogen-containing heterocycle" as used in the present invention relates to monocyclic ring structures having 3 to 8 ring atoms, preferably 5 to 6 ring atoms and comprising at least one nitrogen heteroatom and at least two carbon atoms. Said nitrogen-containing heterocycle includes saturated or unsaturated, substituted or unsubstituted ring systems and may additionally comprise other hetero atom(s) such as sulfur and oxygen.

In a preferred embodiment of the present invention R⁴ and/or R^{4'} is hydrogen.

More specfically, within formula (II) the at least one benzoxazine compound B of the present invention may be embraced by the following structure (B-XIX), 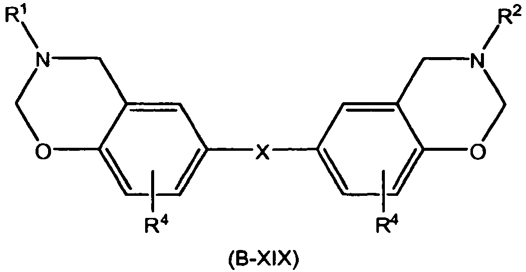 where X is selected from a direct bond, CH₂, C(CH₃)₂, C=O, O, S, S=O and O=S=O, R¹ and R² are the same or different, and R¹ is defined as above and R² is defined as R¹, and R⁴ are the same or different and defined as above.

Representative benzoxazines within structure (B-XIX) include: 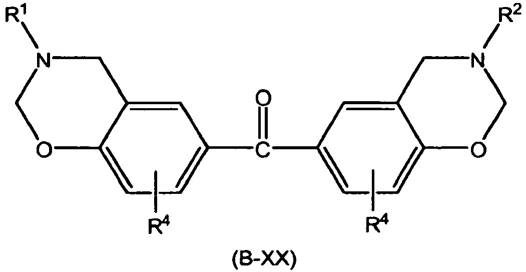 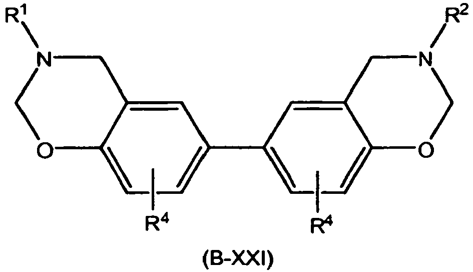 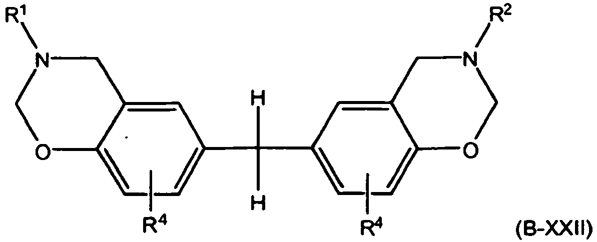 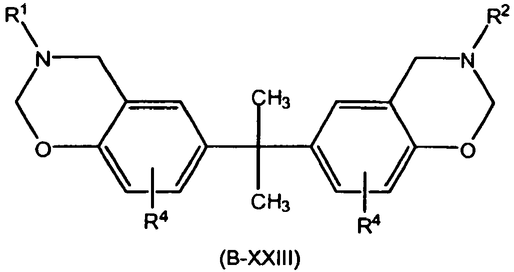 where R¹, R² and R⁴ are as defined above.

Though not embraced by formula (II) and/or formula (IIa) additional benzoxazine compounds B of the present invention are within the following structures: 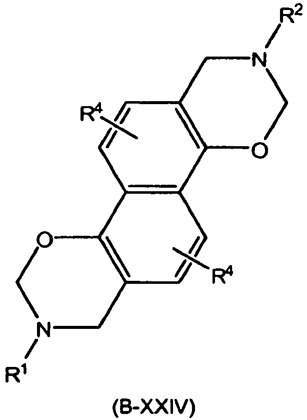 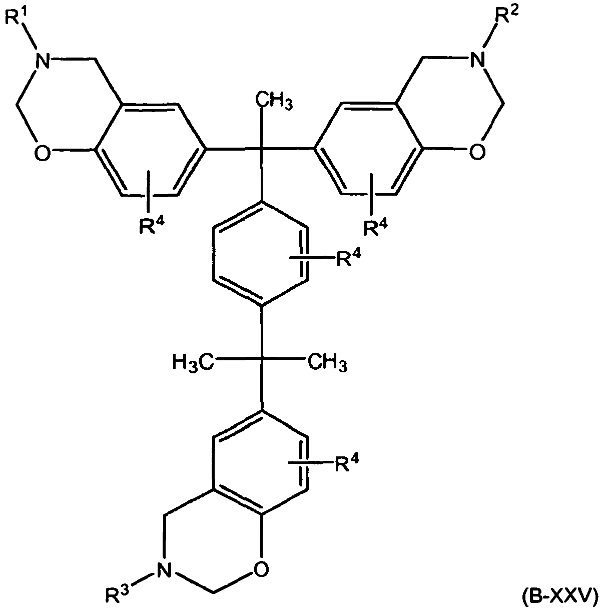 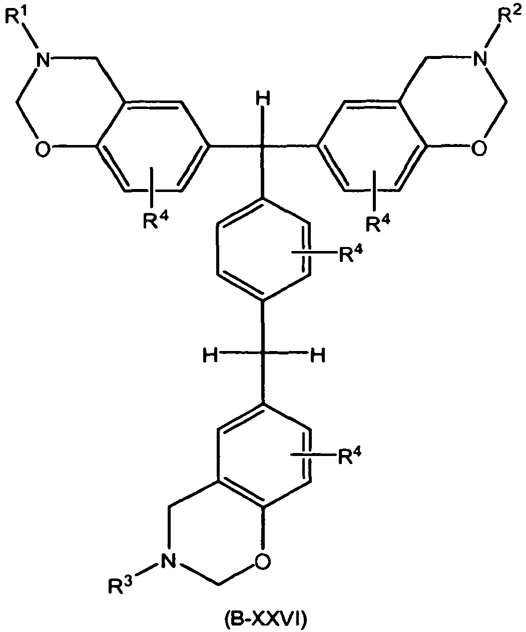 where R¹, R² and R⁴ are as defined above, and R³ is defined as R¹.

Specific examples of the above generically described benzoxazines include:

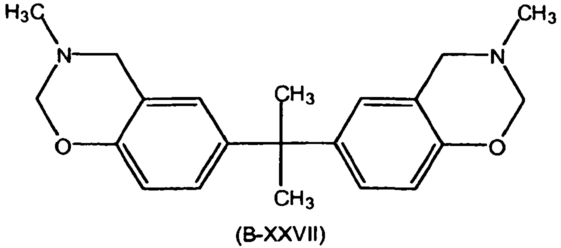

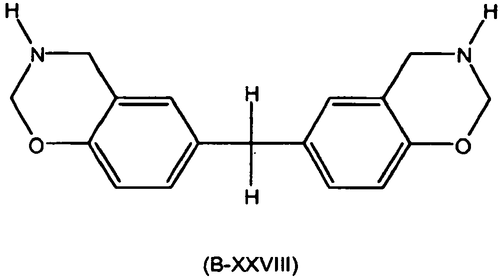

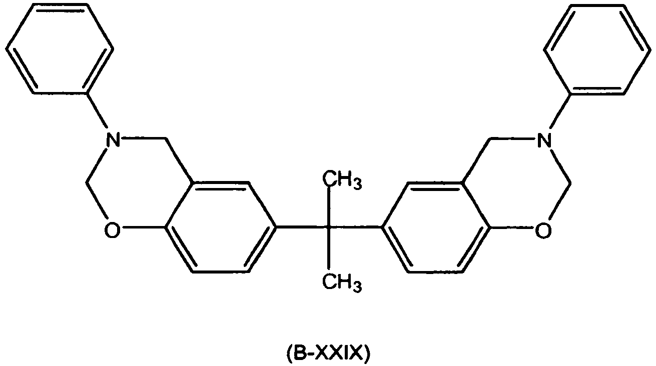

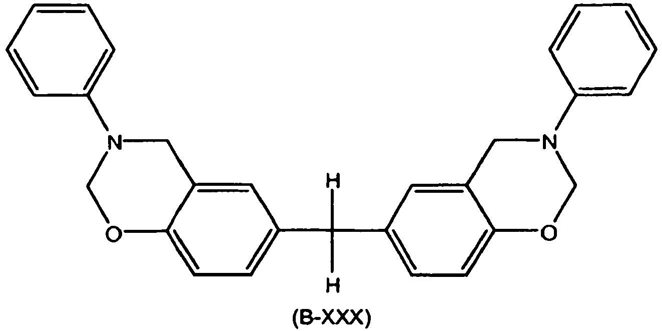

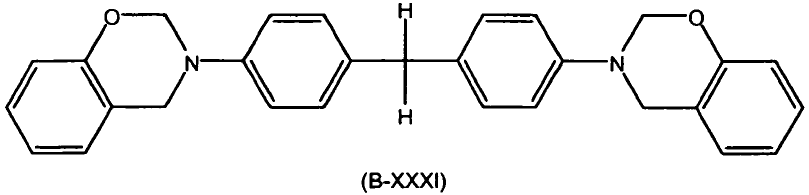

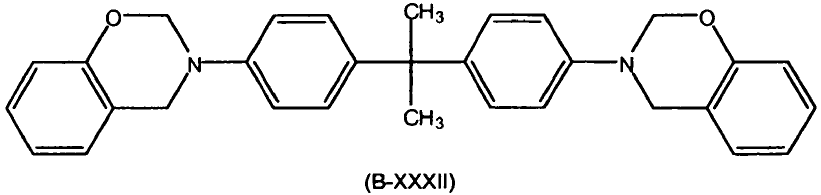

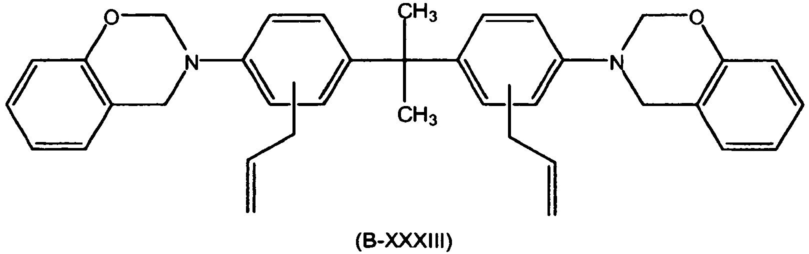

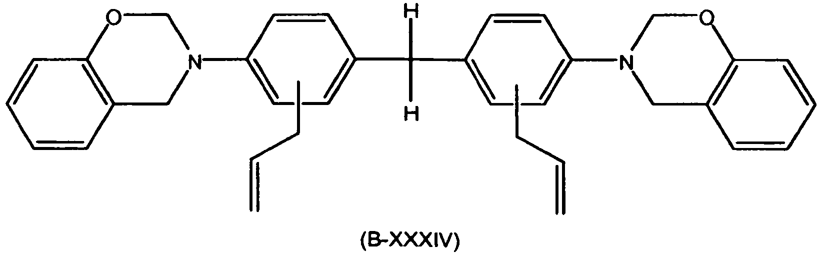

The at least one benzoxazine compound B according to formula (II) and/or formula (IIa) may include the combination of multifunctional benzoxazines and monofunctional benzoxazines, or may be the combination of one or more multifunctional benzoxazines or one or more monofunctional benzoxazines.

Examples of monofunctional benzoxazines may be embraced by the following structure (B-XXXV): 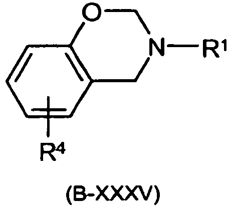 where R¹ and R⁴ are defined as above.

For instance, monofunctional benzoxazines may be embraced by general structure (B-XXXVI), 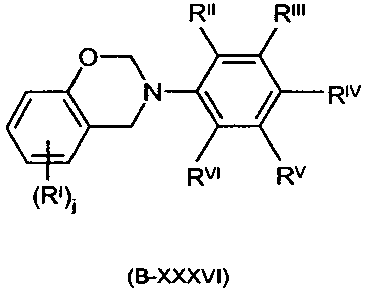 where in this case R¹ is selected from alkyl, alkenyl, each of which being optionally substituted or interrupted by one or more O, N, S, C=O, COO, and NHC=O, and aryl; j is an integer from 0 to 4; and each R^{II}, R^{III}, R^{IV}, R^{V} and R^{VI} does not comprise any hydroxyl group or any nitrogen-containing heterocycle and R^{II}, R^{III}, R^{IV}, R^{V} and R^{VI} are independently selected from hydrogen, alkyl, alkenyl, each of which being optionally substituted or interrupted by one or more S, C=O, and aryl.

Specific examples of such a monofunctional benzoxazine are: 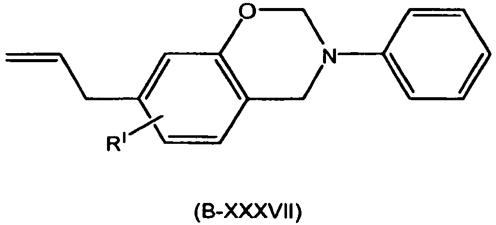 where R^{I} is as defined above; or

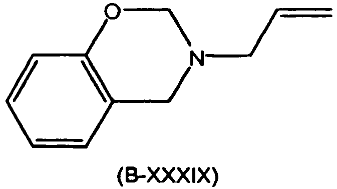

In a particularly preferred embodiment of the present invention the at least one benzoxazine compound B, which is different from benzoxazine compound A, is an "aliphatic benzoxazine", i.e. a benzoxazine having an aliphatic residue bound to the nitrogen atoms of the benzoxazine residue, such as the compound of formula (B-XXVII) above. However in another preferred embodiment it can be desirable to use "aromatic benzoxazines", i.e. benzoxazines having an aromatic residue bound to the nitrogen atoms of the benzoxazine residues such as the compounds of formulas (B-XXIX) or (B-XXX). In some other preferred embodiments mixtures of the before-mentioned benzoxazines are advantageously employed.

Additionally, in some other preferred embodiments of the present invention the polymerizable composition of the present invention may comprise a mixture of at least one benzoxazine according to formula (II) and at least one benzoxazine according to formula (IIa).

Benzoxazines are presently available commercially from several sources, including Huntsman Advanced Materials; Georgia-Pacific Resins, Inc.; and Shikoku Chemicals Corporation, Chiba, Japan.

If desired, however, instead of using commercially available sources, the benzoxazines of the present invention may typically be prepared by reacting a phenolic compound, such as a bisphenol A, bisphenol F, bisphenol S or thiodiphenol, with an aldehyde and an alkyl or aryl amine. U.S. Patent No. 5,543,516, hereby expressly incorporated herein by reference, describes a method of forming benzoxazines, where the reaction time can vary from a few minutes to a few hours, depending on reactant concentration, reactivity and temperature. See generally U.S. Patent Nos. 4,607,091 (Schreiber), 5,021,484 (Schreiber), 5,200,452 (Schreiber) and 5,443,911 (Schreiber).

In a further preferred embodiment of the present invention the polymerizable composition comprises at least one benzoxazine compound A and at least one benzoxazine compound B in a molar ratio from 99.9:0.1 to 0.1:99.9, preferably from 50:50 to 1:99.
Particularly preferred ratios of benzoxazine compound A to benzoxazine compound B are 2:98, 3:97, 4:96, 5:95, 6:94, 7:93, 8:92, 9:91, 10:90, 15:85, 20:80, 25:75, 30:70, 35:65, 40:60 and 45:65.

In certain embodiments of the present invention, the polymerizable composition comprise at least one benzoxazine compound A in an amount from about 5 to about 95 percent by weight, preferably from about 20 to about 80 percent by weight and more preferably from about 40 to 60 percent by weight, based on the total amount of the polymerizable composition.

In further embodiments of the present invention, the polymerizable composition comprises at least one benzoxazine compound B in an amount from about 5 to about 95 percent by weight, preferably from about 20 to about 80 percent by weight and more preferably from about 40 to 60 percent by weight, based on the total amount of the polymerizable composition.

Preferably, the total amount of all benzoxazine compounds in the polymerizable composition is in the range from about 10 to about 100 percent by weight, preferably from about 20 to about 99 percent by weight, and more preferably from about 30 to 95 percent by weight, based on the total amount of the polymerizable composition.

In one embodiment of the present invention the polymerizable composition of the present invention may further comprises one or more epoxy resins, i.e. epoxy-containing compounds even though the addition of epoxy resins is not necessary. Preferably the amount of epoxy resins employed does not exceed 60 wt.-%, more preferably 40 wt.-% and most preferably 10 wt.-%. Particularly preferable are polymerizable compositions that are essentially free of epoxy resins. Commercially available epoxy-containing compounds for use in the polymerizable composition are illustrated below.

The epoxy-containing compounds used may include multifunctional epoxy- containing compounds, such as C₁-C₂₈ alkyl-, poly-phenol glycidyl ethers; polyglycidyl ethers of pyrocatechol, resorcinol, hydroquinone, 4,4'-dihydroxydiphenyl methane (or bisphenol F, such as RE-303-S or RE-404-S available commercially from Nippon Kayuku, Japan), 4,4'-dihydroxy-3,3'-dimethyldiphenyl methane, 4,4'-dihydroxydiphenyl dimethyl methane (or bisphenol A), 4,4'-dihydroxydiphenyl methyl methane, 4,4'-dihydroxydiphenyl cyclohexane, 4,4'-dihydroxy-3,3'-dimethyldiphenyl propane, 4,4'-dihydroxydiphenyl sulfone, and tris(4-hydroxyphenyl) methane; polyglycidyl ethers of transition metal complexes; chlorination and bromination products of the above-mentioned diphenols; polyglycidyl ethers of novolacs; polyglycidyl ethers of diphenols obtained by esterifying ethers of diphenols obtained by esterifying salts of an aromatic hydrocarboxylic acid with a dihaloalkane or dihalogen dialkyl ether; polyglycidyl ethers of polyphenols obtained by condensing phenols and long-chain halogen paraffins containing at least two halogen atoms; phenol novolac epoxy; cresol novolac epoxy; and combinations thereof.

Among the commercially available epoxy-containing compounds suitable for use in the present invention are polyglycidyl derivatives of phenolic compounds, such as those available under the tradenames EPON 825, EPON 826, EPON 828, EPON 1001, EPON 1007 and EPON 1009, cycloaliphatic epoxy-containing compounds such as Araldite CY179 from Huntsman or waterborne dispersions under the tradenames EPI-REZ 3510, EPI-REZ 3515, EPI-REZ 3520, EPI-REZ 3522, EPI-REZ 3540 or EPI-REZ 3546 from Hexion; DER 331, DER 332, DER 383, DER 354, and DER 542 from Dow Chemical Co.; GY285 from Huntsman, Inc.; and BREN-S from Nippon Kayaku, Japan. Other suitable epoxy-containing compounds include polyepoxides prepared from polyols and the like and polyglycidyl derivatives of phenol-formaldehyde novolacs, the latter of which are available commercially under the tradenames DEN 431, DEN 438, and DEN 439 from Dow Chemical Company and a waterborne dispersion ARALDITE PZ 323 from Huntsman.

Cresol analogs are also available commercially such as ECN 1273, ECN 1280, ECN 1285, and ECN 1299 or waterborne dispersions ARALDITE ECN 1400 from Huntsman, Inc. SU-8 and EPI-REZ 5003 are bisphenol A-type epoxy novolacs available from Hexion. Epoxy or phenoxy functional modifiers to improve adhesion, flexibility and toughness, such as the HELOXY brand epoxy modifiers 67, 71, 84, and 505.

Of course, combinations of the different epoxy resins (epoxy-containing compounds) are also desirable for use herein.

The epoxy-containing compounds can be used in the polymerizable composition in an amount of preferably 0 to 60, more preferably 5 to 50 and most preferably 6 to 10 percent by weight based on the total weight of the curable composition.

Further curable ingredients other than benzoxazine-based ingredients and epoxy-based ingredients are for example phenol resins, maleinimide resins, oxazolines, isocyanates and the like.

If desired, reactive diluents, for example styrene oxide, butyl glycidyl ether, 2,2,4-trimethylpentyl glycidyl ether, phenyl glycidyl ether, cresyl glycidyl ether or glycidyl esters of synthetic, highly branched, mainly tertiary, aliphatic monocarboxylic acids, oxazoline group containing compounds may be added to the polymerizable composition to reduce its viscosity.

Other additives which the inventive polymerizable composition can include are tougheners, plasticizers, extenders, microspheres, fillers and reinforcing agents, for example coal tar, bitumen, textile fibers, glass fibers, asbestos fibers, boron fibers, carbon fibers, mineral silicates, mica, powdered quartz, hydrated aluminum oxide, bentonite, wollastonite, kaolin, silica, aerogel or metal powders, for example aluminium powder or iron powder, and also pigments and dyes, such as carbon black, oxide colors and titanium dioxide, fire-retarding agents, thixotropic agents, flow control agents, such as silicones, waxes and stearates, which can, in part, also be used as mold release agents, adhesion promoters, antioxidants and light stabilizers, the particle size and distribution of many of which may be controlled to vary the physical properties and performance of the inventive polymerizable composition.

When used, fillers are used in an amount sufficient to provide the desired rheological properties. Fillers may be used in an amount up to about 50 percent by weight, such as about 5 to about 32 percent by weight, for instance about 10 to about 25 percent by weight.
The fillers may be inorganic ones, such as silicas. For instance, the silica filler may be a silica nanoparticle.

In a preferred embodiment of the present invention the polymerizable composition is cured at temperatures from 20°C to 250°C, preferably from 50°C to 200°C, more preferably from 120°C to 180°C and/or pressures between 1 to 100 atm, preferably between 1 to 5 atm, and more preferably under atmospheric pressure.

The polymerizable composition of the present invention can also be supplemented with additional catalysts without losing their advantages properties in case the use of additional catalysts is desired for specific applications.
In this regard Lewis acids, and other known cationic initiators, such as metal halides; organometallic derivatives; metallophorphyrin compounds such as aluminum phthalocyanine chloride; anhydrides, methyl tosylate, methyl triflate, and triflic acid; and oxyhalides can be added to the polymerizable composition of the present invention.

However taking into account that the above-mentioned catalysts could cause the formation of volatile, toxic and corrosive impurities, polymerizable compositions are preferred that do not comprise the above-mentioned additional catalysts.

As noted, the polymerizable compositions of the present invention are in particular suitable as coatings, adhesives, sealants and matrices for the preparation of reinforced material such as prepregs and towpregs and/or can be used in injection molding or extrusion.
Therefore it is another object of the invention to provide an adhesive, sealant or coating comprising the polymerizable composition of the present invention.

The invention also provides a cured reaction product of the polymerizable composition, in particular cured reaction products containing bundles or layers of fibers infused with the polymerizable composition, and a method of preparing such material.

In this regard, the invention relates to processes for producing a prepreg or a towpregs. One such process includes the steps of (a) providing a layer or bundle of fibers; (b) providing a polymerizable composition of the present invention; (c) joining said polymerizable composition and the layer or bundle of fibers to form a prepreg or a towpregs assembly; and (d) optionally removing excess polymerizable composition from the prepreg or towpreg assembly, and exposing the resulting prepreg or towpreg assembly to elevated temperature and pressure conditions sufficient to infuse the layer or bundle of fibers with the polymerizable composition to form a prepreg or a towpregs assembly as the cured reaction product.

Another such process for producing a prepreg or towpreg, includes the steps of (a) providing a layer or bundle of fibers; (b) providing a polymerizable composition of the present invention in liquid form; (c) passing the layer or bundle of fibers through said polymerizable composition to infuse the layer or bundle of fibers with said polymerizable composition; and (d) removing excess of said polymerizable composition from the prepreg or towpreg assembly, and exposing the resulting prepreg or towpreg assembly to elevated temperature and pressure conditions sufficient to infuse the layer or bundle of fibers with the polymerizable composition to form a prepreg or a towpregs assembly as the cured reaction product.

Generally, the fiber layer or bundle may be constructed from unidirectional fibers, woven fibers, chopped fibers, non-woven fibers or long, discontinuous fibers.

The fiber chosen may be selected from carbon, glass, aramid, boron, polyalkylene, quartz, polybenzimidazole, polyetheretherketone, polyphenylene sulfide, poly p-phenylene benzobisoaxazole, silicon carbide, phenotformaldehyde, phthalate and napthenoate.

The carbon is selected from polyacrylonitrile, pitch and acrylic, and the glass is selected from S glass, S2 glass, E glass, R glass, A glass, AR glass, C glass, D glass, ECR glass, glass filament, staple glass, T glass and zirconium oxide glass.

The inventive polymerizable composition (and prepregs and towpregs prepared therefrom) is particularly useful in the manufacture and assembly of composite parts for aerospace and industrial end uses, bonding of composite and metal parts, core and core-fill for sandwich structures and composite surfacing.

The inventive polymerizable composition is also useful as a coating, sealant or adhesive for the electronics industry. Suitable substrates on which the polymerizable compositions of the present invention are applied are metals such as steel, aluminum, titanium, magnesium, brass, stainless steel, galvanized steel, like HDG-steel and EG-steel; silicates such as glass and quartz; metal oxides; concrete; wood; electronic chip material, for instance semiconductor chip material; or polymers such as polyimide films and polycarbonate.

The inventors of the present invention surprisingly found that by adding at least one benzoxazine compound A to the polymerizable composition of the present invention the polymerization rate of the inventive polymerizable composition can be increased at temperatures up to 250°C.

In this regard the present invention relates to the use of at least one benzoxazine compound according to formula (I), 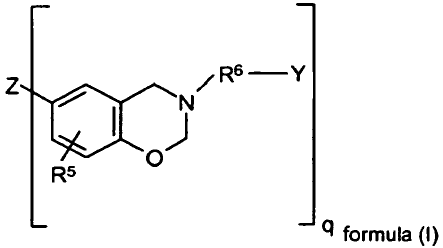 wherein q is an integer from 1 to 4, Z is selected from the group consisting of a direct bond (when q is 2), hydrogen (when q is 1), alkyl (when q is 1), alkylene (when q is 2 to 4), carbonyl (when q is 2), oxygen (when q is 2), thiol (when q is 1), sulfur (when q is 2), sulfoxide (when q is 2), and sulfone (when q is 2), Y is selected from the group consisting of a hydroxyl group, and a nitrogen-containing heterocycle, R⁶ is a linear or branched divalent alkylene group, comprising 1 to 15 carbon atoms, which may be interrupted by one or more heteroatom(s), selected from oxygen, nitrogen and sulfur and R⁵ is selected from hydrogen, halogen, alkyl, alkenyl or R⁵ is a divalent residue creating a naphthoxazine residue out of the benzoxazine structure, as a curative for curable compositions.

The invention also relates to a method to increase the polymerization rate of a polymerizable composition at temperatures up to 250°C. Said method includes the of steps of
a) adding at least one benzoxazine compound according to formula (I), 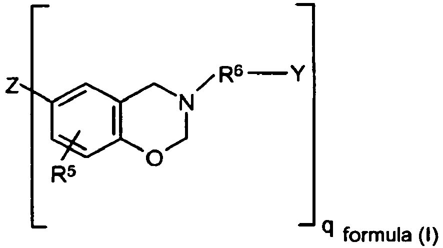 wherein q is an integer from 1 to 4, Z is selected from the group consisting of a direct bond (when q is 2), hydrogen (when q is 1), alkyl (when q is 1), alkylene (when q is 2 to 4), carbonyl (when q is 2), oxygen (when q is 2), thiol (when q is 1), sulfur (when q is 2), sulfoxide (when q is 2), and sulfone (when q is 2), Y is selected from the group consisting of a hydroxyl group, and a nitrogen-containing heterocycle, R⁶ is a linear or branched divalent alkylene group, comprising 1 to 15 carbon atoms, which may be interrupted by one or more heteroatom(s), selected from oxygen, nitrogen and sulfur and R⁵ is selected from hydrogen, halogen, alkyl, alkenyl or R⁵ is a divalent residue creating a naphthoxazine residue out of the benzoxazine structure, to a curable composition;
b) subjecting the curable composition to conditions appropriate to cure the curable composition.

The term "polymerization rate" as used in the present invention means an average value of the amounts of a change in polymerization conversion per every unit hour (%/hour) obtained in the first 4 hours after starting the polymerization. The polymerization rate can easily be determined by a man skilled in the art using known techniques, such as GC-analysis, NMR- or IR spectroscopy.

In preferred embodiments of the present invention the polymerization rate is determined at temperatures from 20°C to 250°C, preferably from 50°C to 200°C, and more preferably from 120°C to 180°C and/or pressures between 1 to 100 atm, preferably between 1 to 5 atm, and more preferably under atmospheric pressure.

The term curable composition, as used in the present invention, preferably refers to a composition which comprises at least one benzoxazine compound B.

In a particular preferred embodiment of the present invention the curable composition comprises at least one benzoxazine compound B in an amount from about 5 to about 100 percent by weight, preferably from about 20 to about 99 percent by weight and more preferably from about 40 to about 95 percent by weight, based on the total amount of the curable composition

Preferably, the curable composition of the present invention is cured at temperatures from 20°C to 250°C. preferably from 50°C to 200°C, and more preferably from 120°C to 180°C and/or pressures between 1 to 100 atm, preferably between 1 to 5 atm, and more preferably under atmospheric pressure.

Under comparable reaction conditions, the polymerization rate of curable compositions is significantly increased by adding at least one benzoxazine compound according to formula (I) to the curable composition of the present invention. In particular curable compositions can be cured in an environmentally friendly process at relatively low temperatures in short time periods by using at least one benzoxazine compound according to formula (I) as a curative.

Due to the high reactivity of the curable composition of the present invention, the amount of thermal energy used in the polymerization process can be reduced. Additionally, the amount of additional catalysts used in the polymerization process, such as strong acids and/or Lewis acids can be reduced. Therefore the polymerization process can be carried out without caution to decomposition of said catalysts and resulting evolution of toxic and/or corrosive by-products.

The invention is further illustrated by the following examples.

### Examples

### A. Synthesis of functionalized benzoxazines

### A.1 Synthesis of hydroxyl-functionalized benzoxazines

As noted, hydroxyl-functionalized benzoxazines (Y = OH) according to formula (I) can be prepared according to any method as e.g. the method disclosed in the Japanese patent application JP 2002-302486 A on page 11, line 66-100. The method relies on the reaction of a phenolic compound, with an aldehyde, such as formaldehyde and aliphatic amino alcohol. The reaction time can vary from a few minutes to a few hours, depending on reactant concentration, reactivity and temperature. Alternatively, a method for preparing the hydroxyl-functionalized benzoxazines according to formula (I) is disclosed by Kiskan and Yagci in Polymer 46 (2005), pp 11690 - 11697 and by Kiskan, Yagci and Ishida in Journal of Polymer Science: Part A: Polymer Chemistry (2008), vol. 46, pp 414- 420.

#Box-1, #Box-2, and Ref-Box can be prepared according to any of the above-described methods by reacting monoethanolamine and formaldehyde with 4-methyl-phenol (#Box-1), monoethanolamine and formaldehyde with Bisphenol A (#Box-2), 2-(2-aminoethoxy)ethanol, and methylamine and formaldehyde with 4-methyl-phenol (Ref-Box).

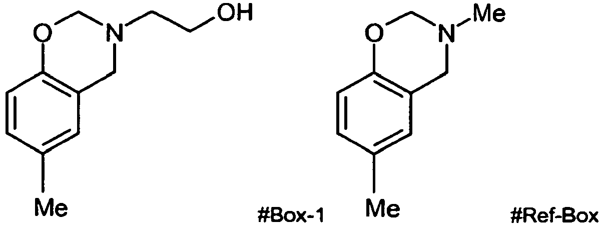

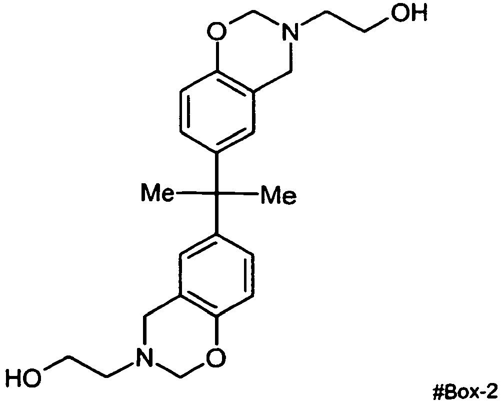

### A.2 Synthesis of heterocyclic-functionalized benzoxazines

### A.2.1 Synthesis of #Box-4

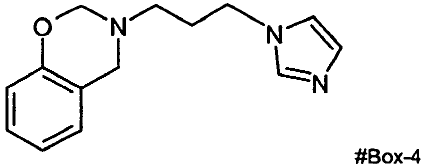

In a 500 ml three-necked round bottom flask equipped with stirrer, condenser, electric thermometer, dropping funnel and nitrogen gas inlet a solution of 50.07 g N-(3-aminopropyl)-imidazole (0.4 mol, 1.0 eq.) in ethyl acetate (140 ml) was added dropwise within 40 minutes at 5.0 to 12.7°C to 64.93 g formaldehyde solution (37 % in water, 2.0 eq.) in ethyl acetate (50 ml). Under a nitrogen gas atmosphere a solution of 37.64 g phenol (0.4 mol, 1.0 eq.) in ethyl acetate (120 ml) was added dropwise within 10 minutes at 7.8 to 9.8°C to the reaction mixture. The reaction mixture was subsequently heated and maintained under reflux conditions for 5.5 hours while stirring.
The light-hazy solution resulting from the above reaction was washed several times with a solution of NaCl and NaOH and several times with 10% ethanol in water. The organic phase was then dried over sodium sulphate and concentrated using a rotary evaporator. Remaining solvents were removed under reduced pressure.

27.8 g of product were obtained, corresponding to 28.5% of the theoretical yield.

### B Curing experiments

### B.1 Curing of hydroxyl-functionalized benzoxazines

An amount of 5.0 g #Box-1 (0.026mol) was divided into ten portions (500 mg each) and each portion was placed in a test tube. To the resulting 10 test tubes Argon inlets were attached and the test tubes were heated in an oil bath at 150°C. From time to time, test tubes were taken away from the oil bath one-by-one, and each portion was analyzed by GC to determine conversions of #Box-1.

Following the same procedure the conversion of different para-methyl-substituted non-hydroxyl-functionalized benzoxazines (R = Me (Ref-Box), n-Pr, -(CH₂)₂-OMe), referential examples) were determined at 150°C.

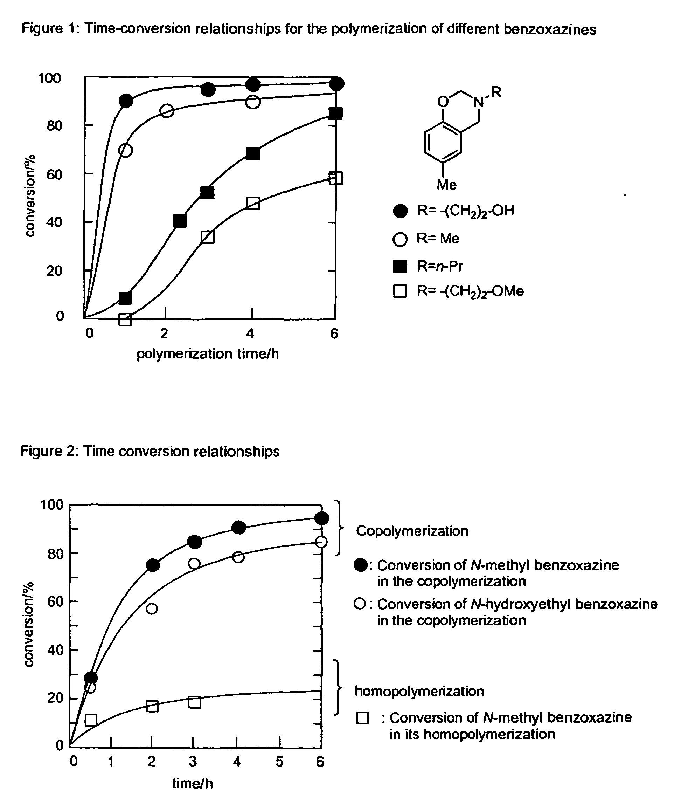

The resulting time-conversion relationships are shown in Table 1 and visualized in Figure 1.

**Table 1: Time-conversion relationships for the polymerization of different benzoxazines**

| Time (h) | 1 | 2 | 3 | 4 | 6 |
|---|---|---|---|---|---|
| Conversion (%) #Box-1 | 92 | - | 94 | 95 | 98 |
| Conversion (%) R= Me (Ref-Box) (Ref.) | 69 | 85 | - | 88 | - |
| Conversion (%) R= n-Pr (Ref.) | 9 | - | 56 | 72 | 86 |
| Conversion (%) R= -(CH₂)₂-OMe (Ref.) | 2 | - | 34 | 46 | 58 |

As shown in Table 1 and visualized in Figure 1, the hydroxyl-functionalized benzoxazine #Box-1 shows the highest polymerization rate. At 1 h, about 90% of #Box-1 is consumed, compared to only 69% of Ref-Box.

### B.2 Curing of polymerizable compositions comprising hydroxyl-functionalized benzoxazines

A 1:1 mixture of #Box-1 (2.51g, 0.013mol) and Ref-Box (2.12g, 0.013mol) was divided into ten portions and each portion was placed in a test tube. To the resulting 10 test tubes Argon inlets were attached and the test tubes were heated in an oil bath at 120°C. From time to time, test tubes were taken away from the oil bath one-by-one, and each portion was analyzed by GC to determine conversions of #Box-1 and Ref-Box.

Following the same procedure the conversion of Ref-Box (referential example) in its homopolymerization was determined at 120°C.

The resulting time-conversion relationships are shown in Table 2 and visualized in Figure 2.

**Table 2: Time-conversion relationships**

| Time (h) | 0.5 | 2 | 3 | 4 | 6 |
|---|---|---|---|---|---|
| Conversion (%) Ref-Box in the copolymerization | 29 | 75 | 85 | 91 | 95 |
| Conversion (%) #Box-1 in the copolymerization | 25 | 57 | 76 | 79 | 85 |
| Conversion (%) Ref-Box in its homopolymerization (Ref.) | 12 | 17 | 19 | 20 | 39 |

Figure 2 and Table 2 indicate that the addition of hydroxyl-functionalized #Box-1 leads to a significant acceleration of the copolymerization rate of Ref-Box. As a result, both benzoxazines can be polymerized (at a relatively high polymerization rate) at 120°C in the absence of cationic catalysts.

### B.3 Curing of polymerizable compositions comprising hydroxyl-functionalized benzoxazines and bifunctional benzoxazines

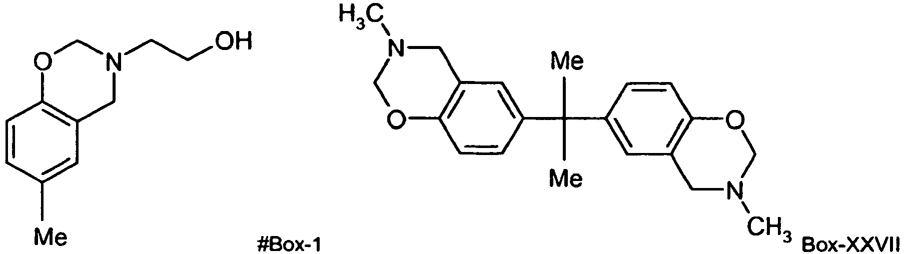

A 1:2 mixture of #Box-1(50mg, 1.48*10⁻⁴ mol) and Box-XXVII (57.2 mg, 2.96*10⁻⁴ mol) was placed in an aluminum pot. This pot was set in a TMA instrument and heated at 150°C. The increase in storage modulus (E') during the curing reaction was monitored at 150°C by TMA (thermo mechanical analysis). The resulting time-E' relationship exhibited an inflection point. The time at which the inflection is observed is defined as the "gelation point (T_{gel})" Different gelation points are shown in Table 3.

### Referential example

Following the same procedure the curing reaction of a 1:2 mixture of Ref-Box (50 mg, 1.48*10⁻⁴ mol) and Box-XXVII (48.3 mg, 2.96*10⁻⁴ mol) was monitored at 150°C by TMA (thermomechanical analysis). The resulting time-E' relationship exhibited an inflection point. The time at which the inflection is observed is defined as the "gelation point (T_{gel})".

**Table 3: Gelation points**

| Combination | Gelation point T_{gel} [min] |
|---|---|
| Box-XVII + #Box-1 | 49 |
| Box-XXVII + Ref-Box (Ref.) | 88 |

Table 3 indicates that the addition of the hydroxyl-functionalized #Box-1 leads to a significant acceleration of the polymerization rate of the bifunctional benzoxazines Box-XXVII.

### B.4 Curing of bifunctional benzoxazines

#Box-2(100 mg, 2.51*10⁻⁴ mol) is placed in an aluminum pot. The pot was set in the TMA instrument and heated at 150°C. The increase in storage modulus (E') during the curing reaction was monitored at 150°C by TMA (thermomechanical analysis). The resulting time-E' relationship exhibited an inflection point. The time at which the inflection is observed is defined as the "gelation point (T_{gel})". Different gelation points are shown in Table 4.

Following the same procedure the curing reaction of Box-XXVII (100 mg, 2.95*10⁻⁴ mo) was monitored at 150°C by TMA (thermomechanical analysis). The resulting time-E' relationship exhibited an inflection point. The time at which the inflection is observed is defined as the "gelation point (T_{gel})".

**Table 4: Gelation points**

| Compound | Gelation time T_{gel} [min] |
|---|---|
| #Box-2 | 61 |
| Box-XXVII | 118 |

As shown in Table 4, the polymerization rate of the hydroxyl-functionalized benzoxazine #Box-2 is much higher than the polymerization rate of the aliphatic benzoxazine Box-XXVII.

### B.5 Curing of polymerizable compositions comprising heterocyclic-functionalized benzoxazines

### B.5.1

The polymerizable composition described in the following example contains the following benzoxazine (Box-XXXI):

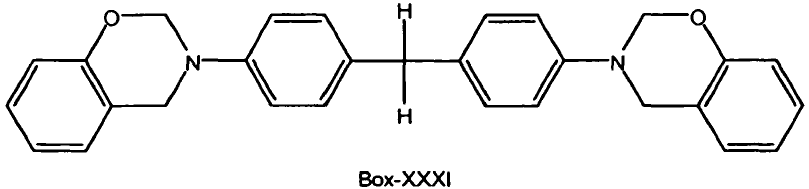

To 95.0 g of the above shown benzoxazine Box-XXXI, 5.0 g of the imidazole-functionalized benzoxazine #Box-4 were added. The mixture was stirred under vacuo (< 1 mbar) for 15 to 30 minutes at a temperature of 105 to 115 °C. The thus prepared polymerizable composition was stored at room temperature in a sealed container.

The curing reaction of the polymerizable composition was monitored by DSC (differential scanning calorimetry) using the following conditions:
Atmosphere Nitrogen stream
Heating rate 10° C/min
Cooling rate 10° C/min
Temperature range 50 to 300°C
Amount of sample 6 to 7 mg
Sample container Aluminum container with 5 holes

### Referential example

The curing reaction (homopolymerization) of benzoxazine Box-XXXI was monitored by DSC (differential scanning calorimetry) using the same conditions as above:

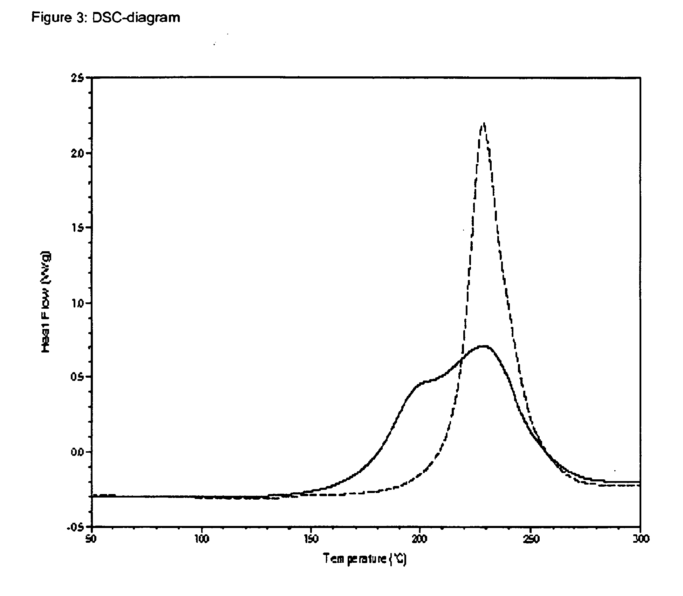

Figure 3 shows the following DSC-diagrams:
   Solid line: polymerizable composition, comprising 95 wt.-% Box-XXXI
   and 5 wt.-% #-Box-4 (invention);
   Dotted line: composition, comprising 100 wt.-% Box-XXXI (referential example).

The DSC-diagrams of Figure 3 clearly indicate that the curing temperature of a benzoxazine-based polymerizable composition can significantly be reduced, by adding the imidazole-functionalized benzoxazine #Box-4.

### B.5.2

The polymerizable composition described in the following example contains the following benzoxazine (Box-XXVII):

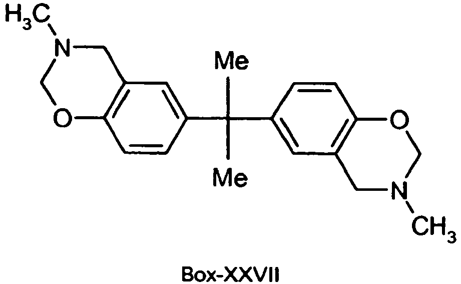

To 97.5 g of the above shown benzoxazine Box-XXVII, 2.5 g of the imidazole-functionalized benzoxazine #Box-4 were added. The mixture was stirred under vacuo (< 1 mbar) for 15 to 30 minutes at a temperature of 105 to 115 °C. The thus prepared polymerizable composition was stored at room temperature in a sealed container.

The curing reaction of the polymerizable composition was monitored by DSC (differential scanning calorimetry) using the following conditions:
Atmosphere Nitrogen stream
Heating rate 10° C/min
Cooling rate 10° C/min
Temperature range 50 to 300°C
Amount of sample 6 to 7 mg
Sample container Aluminum container with 5 holes

### Referential example

The curing reaction (homopolymerization) of benzoxazine Box-XXVII was monitored by DSC (differential scanning calorimetry) using the same conditions as above:

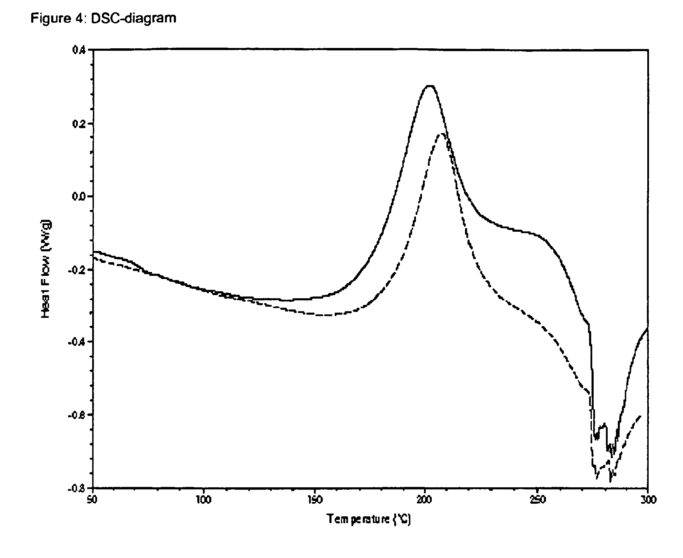

Figure 4 shows the following DSC-diagrams:
   Solid line: polymerizable composition, comprising 97.5 wt.-% Box-XXVII
   and 2.5 wt.-% #-Box-4 (invention);
   Dotted line: composition, comprising 100 wt.-% Box-XXVII (referential example).

The DSC-diagrams of Figure 4 clearly indicate that the curing temperature of a benzoxazine-based polymerizable composition can significantly be reduced, by adding the imidazole-functionalized benzoxazine #Box-4.

## Claims

1. Polymerizable composition, comprising
a) at least one benzoxazine compound A according to formula (I), 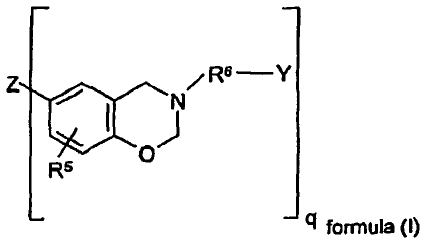 wherein q is an integer from 1 to 4, Z is selected from the group consisting of a direct bond (when q is 2), hydrogen (when q is 1), alkyl (when q is 1), alkylene (when q is 2 to 4), carbonyl (when q is 2), oxygen (when q is 2), thiol (when q is 1), sulfur (when q is 2), sulfoxide (when q is 2), and sulfone (when q is 2), Y is selected from the group consisting of a hydroxyl group and a nitrogen-containing heterocycle selected from substituted or unsubstituted imidazoles, R⁶ is a linear or branched divalent alkylene group, comprising 1 to 15 carbon atoms, which may be interrupted by one or more heteroatom(s), selected from oxygen, nitrogen and sulfur and R⁵ is selected from hydrogen, halogen, alkyl, alkenyl or R⁵ is a divalent residue creating a naphthoxazine residue out of the benzoxazine structure, and
b) at least one benzoxazine compound B, which does not correspond to formula (Ia), 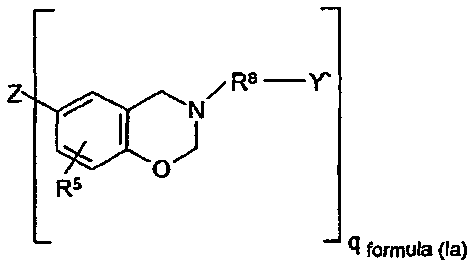 wherein q, Z, R⁵ and R⁶ are defined as above and Y' is selected from the group consisting of a hydroxyl group and a nitrogen-containing heterocycle.

2. Polymerizable composition according to claim 1, wherein R⁶ is a linear divalent alkylene group comprising 1 to 4 carbon atoms.

3. Polymerizable composition according to Claim 1, wherein R⁶ Is a linear divalent alkylene group, which Is interrupted by at least one oxygen atom.

4. Polymerizable composition according to any of claims 1 to 3, wherein Y is a hydroxyl group.

5. Polymerizable composition according to any of claims 1 to 4, wherein the benzoxazine compound B comprises at least one benzoxazine compound according to formula (II) or formula (IIa), 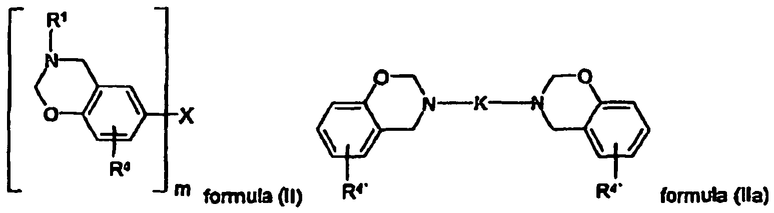 wherein m is an integer from 1 to 4, X Is selected from the group consisting of a direct bond (when m is 2), hydrogen (when m is 1), alkyl (when m is 1), alkylene (when m is 2 to 4), carbonyl (when m is 2), oxygen (when m is 2), thiol (When m is 1), sulfur (when m is 2), sulfoxide (when m Is 2), and sulfone (when m Is 2), R¹ is selected from hydrogen, alkyl, alkenyl and aryl and R¹ does not comprise any hydroxyl group or any nitrogen-containing heterocycle, K is selected from the group consisting of biphenyl, diphenyl methane, diphenyl isopropane, diphenyl sulfide, diphenyl sulfoxide, diphenyl sulfone, and diphenyl ketone, and R⁴ is selected from hydrogen, halogen, alkyl, alkenyl or R⁴ is a divalent residue creating a naphthoxazine residue out of the benzoxazine structure and R⁴ is defined as R⁴.

6. Polymerizable composition according to any of claims 1 to 6, wherein said polymerizable composition comprises at least one benzoxazine compound A and at least one benzoxazines compound B In a molar ratio from 99.9:0.1: to 0.1:99.9, preferably from 50:50 to 1:99.

7. Polymerizable composition according to any of claims 1 to 6, wherein the total amount of all benzoxazine compounds in the polymerizable composition is in the range from about 10 to about 100 percent by weight, based on the total amount of the polymerizable composition.

8. Polymerizable composition according to any of claims 1 to 7, wherein said polymerizable composition comprises at least one epoxy component.

9. Polymerizable composition according to any of claims 1 to 8, wherein said polymerizable composition is cured at temperatures from 20°C to 250°C, preferably from 50°C to 200°C, and more preferably from 120°C to 180°C.

10. A cured reaction product of the polymerizable composition according to any of claims 1 to 9.

11. The cured reaction product according to claim 10, comprising a layer or bundle of fibers infused with the polymerizable composition according to any of claims 1 to 9 before curing.

12. A process for producing the cured reaction product of claim 11, steps of which comprise:
a) providing a layer or bundle of fibers;
b) providing the polymerizable composition according to any of claims 1 to 9;
c) joining the polymerizable composition and the layer or bundle of fibers to form a prepreg or a towpreg assembly; and
d) optionally removing excess polymerizable composition from the prepreg or towpreg assembly, and
exposing the resulting prepreg or towpreg assembly to elevated temperature and pressure conditions sufficient to infuse the layer or bundle of fibers with the polymerizable composition to form the cured reaction product.

13. An adhesive, sealant or coating composition, comprising the polymerizable composition according to any of claims 1 to 9.

14. Use of the at least one benzoxazine compound according to formula (I), 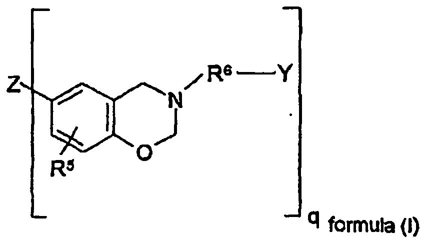 wherein q is an integer from 1 to 4, Z is selected from the group consisting of a direct bond (when q is 2), hydrogen (when q is 1), alkyl (when q is 1), alkylene (when q is 2 to 4), carbonyl (when q is 2), oxygen (when q is 2), thiol (when q is 1), sulfur (when q is 2), sulfoxide (when q is 2), and sulfone (when q is 2), Y is selected from the group consisting of a hydroxyl group, and a nitrogen-containing heterocycle selected from substituted or unsubstituted imidazoles, R⁶ is a linear or branched divalent alkylene group, comprising 1 to 15 carbon atoms, which may be interrupted by one or more heteroatom(s), selected from oxygen, nitrogen and sulfur and R⁵ is selected from hydrogen, halogen, alkyl, alkenyl or R⁵ is a divalent residue creating a naphthoxazine residue out of the benzoxazine structure, as a curative for curable compositions.

15. A method to increase the polymerization rate of a curable composition at temperatures up to 250°C, steps of which comprise:
a) adding at least one benzoxazine compound according to formula (I), 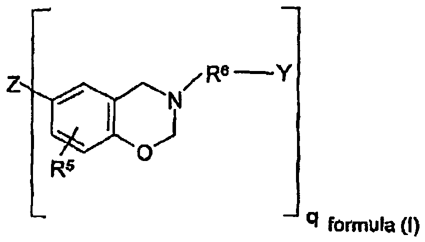 wherein q is an integer from 1 to 4, Z is selected from the group consisting of a direct bond (when q is 2), hydrogen (when q is 1), alkyl (when q is 1), alkylene (when q is 2 to 4), carbonyl (when q is 2), oxygen (when q is 2), thiol (when q is 1), sulfur (when q is 2), sulfoxide (when q is 2), and sulfone (when q is 2), Y is selected from the group consisting of a hydroxyl group, and a nitrogen-containing heterocycle, selected from substituted or unsubstituted imidazoles, R⁶ is a linear or branched divalent alkylene group, comprising 1 to 15 carbon atoms, which may be interrupted by one or more heteroatom(s), selected from oxygen, nitrogen and sulfur and R⁵ is selected from hydrogen, halogen, alkyl, alkenyl or R⁵ is a divalent residue creating a naphthoxazine residue out of the benzoxazine structure, to a curable composition;
b)subjecting the curable composition to conditions appropriate to cure the curable composition.

## Patentansprüche

1. Polymerisierbare Zusammensetzung, umfassend
a) mindestens eine Benzoxazinverbindung A gemäß der Formel (I), 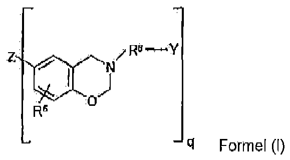 wobei q eine ganze Zahl von 1 bis 4 ist, Z aus der Gruppe bestehend aus einer direkten Bindung (wenn q 2 ist), Wasserstoff (wenn q 1 ist), Alkyl (wenn q 1 ist), Alkylen (wenn q 2 bis 4 ist), Carbonyl (wenn q 2 ist), Sauerstoff (wenn q 2 ist), Thiol (wenn q 1 ist), Schwefel (wenn q 2 ist), Sulfoxid (wenn q 2 ist) und Sulfon (wenn q 2 ist) ausgewählt ist, Y aus der Gruppe bestehend aus einer Hydroxylgruppe und einem aus substituierten oder unsubstituierten Imidazolen ausgewählten stickstoffhaltigen Heterocyclus ausgewählt ist, R⁶ eine lineare oder verzweigte zweiwertige, 1 bis 15 Kohlenstoffatome umfassende Alkylengruppe ist, welche durch ein oder mehrere aus Sauerstoff, Stickstoff und Schwefel ausgewählte Heteroatome unterbrochen sein kann, und R⁵ aus Wasserstoff, Halogen, Alkyl, Alkenyl ausgewählt ist oder R⁵ ein zweiwertiger Rest ist, der einen Naphthoxazinrest aus der Benzoxazinstruktur bildet, und
b) mindestens eine Benzoxazinverbindung B, welche nicht der Formel (Ia) entspricht, 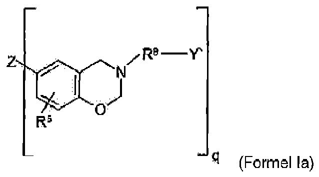 wobei q, Z, R⁵ und R⁶ wie oben definiert sind und Y' aus der Gruppe bestehend aus einer Hydroxylgruppe und einem stickstoffhaltigen Heterocyclus ausgewählt ist.

2. Polymerisierbare Zusammensetzung nach Anspruch 1, wobei R⁶ eine lineare zweiwertige Alkylengruppe ist, die 1 bis 4 Kohlenstoffatome umfasst.

3. Polymerisierbare Zusammensetzung nach Anspruch 1, wobei R⁶ eine lineare zweiwertige Alkylengruppe ist, welche durch mindestens ein Sauerstoffatom unterbrochen ist.

4. Polymerisierbare Zusammensetzung nach irgendeinem der Ansprüche 1 bis 3, wobei Y eine Hydroxylgruppe ist.

5. Polymerisierbare Zusammensetzung nach irgendeinem der Ansprüche 1 bis 4, wobei die Benzoxazinverbindung B mindestens eine Benzoxazinverbindung gemäß der Formel (II) oder Formel (IIa) umfasst, 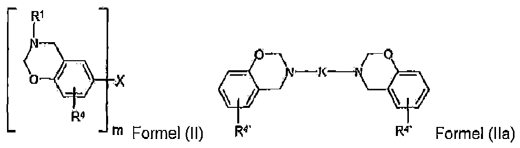 wobei m eine ganze Zahl von 1 bis 4 ist, X aus der Gruppe bestehend aus einer direkten Bindung (wenn m 2 ist), Wasserstoff (wenn m 1 ist), Alkyl (wenn m 1 ist), Alkylen (wenn m 2 bis 4 ist), Carbonyl (wenn m 2 ist), Sauerstoff (wenn m 2 ist), Thiol (wenn m 1 ist), Schwefel (wenn m 2 ist), Sulfoxid (wenn m 2 ist) und Sulfon (wenn m 2 ist) ausgewählt ist, R¹ aus Wasserstoff, Alkyl, Alkenyl und Aryl ausgewählt ist und R¹ nicht irgendeine Hydroxylgruppe oder irgendeinen stickstoffhaltigen Heterocyclus umfasst, K aus der Gruppe bestehend aus Biphenyl, Diphenylmethan, Diphenylisopropan, Diphenylsulfid, Diphenylsulfoxid, Diphenylsulfon und Diphenylketon ausgewählt ist und R⁴ aus Wasserstoff, Halogen, Alkyl, Alkenyl ausgewählt ist oder R⁴ ein zweiwertiger Rest ist, der einen Naphthoxazinrest aus der Benzoxazinstruktur bildet, und R^{4'} wie R⁴ definiert ist.

6. Polymerisierbare Zusammensetzung nach irgendeinem der Ansprüche 1 bis 5, wobei die polymerisierbare Zusammensetzung mindestens eine Benzoxazinverbindung A und mindestens eine Benzoxazinverbindung B in einem Molverhältnis von 99,9:0,1 bis 0,1:99,9, vorzugsweise 50:50 bis 1:99, umfasst.

7. Polymerisierbare Zusammensetzung nach irgendeinem der Ansprüche 1 bis 6, wobei die Gesamtmenge aller Benzoxazinverbindungen in der polymerisierbaren Zusammensetzung im Bereich von ungefähr 10 bis ungefähr 100 Gewichtsprozent bezogen auf die Gesamtmenge der polymerisierbaren Zusammensetzung liegt.

8. Polymerisierbare Zusammensetzung nach irgendeinem der Ansprüche 1 bis 7, wobei die polymerisierbare Zusammensetzung mindestens eine E-poxidkomponente umfasst.

9. Polymerisierbare Zusammensetzung nach irgendeinem der Ansprüche 1 bis 8, wobei die polymerisierbare Zusammensetzung bei Temperaturen von 20 °C bis 250 °C, vorzugsweise 50 °C bis 200 °C und bevorzugter 120 °C bis 180 °C gehärtet wird.

10. Gehärtetes Reaktionsprodukt der polymerisierbaren Zusammensetzung nach irgendeinem der Ansprüche 1 bis 9.

11. Gehärtetes Reaktionsprodukt nach Anspruch 10, umfassend eine Faserschicht oder ein Faserbündel, die/das vor dem Härten mit der polymerisierbaren Zusammensetzung nach irgendeinem der Ansprüche 1 bis 9 getränkt wird,

12. Verfahren zur Herstellung des gehärteten Reaktionsprodukts nach Anspruch 11, dessen Schritte Folgendes umfassen:
a) Bereitstellen einer Faserschicht oder eines Faserbündels;
b) Bereitstellen der polymerisierbaren Zusammensetzung nach irgendeinem der Ansprüche 1 bis 9;
c) Verbinden der polymerisierbaren Zusammensetzung und der Faserschicht oder des Faserbündels, um eine Prepreg- oder Towpreg-Anordnung zu bilden; und
d) gegebenenfalls Entfernen von überschüssiger polymerisierbarer Zusammensetzung aus der Prepreg- oder Towpreg-Anordnung, und
Aussetzen der resultierenden Prepreg- oder Towpreg-Anordnung erhöhten Temperatur- und Druckbedingungen, die ausreichen, um die Faserschicht oder das Faserbündel mit der polymerisierbaren Zusammensetzung zu tränken, um das gehärtete Reaktionsprodukt zu bilden.

13. Klebstoff-, Dichtstoff- oder Beschichtungszusammensetzung, umfassend die polymerisierbare Zusammensetzung nach irgendeinem der Ansprüche 1 bis 9.

14. Verwendung der mindestens einen Benzoxazinverbindung gemäß der Formel (I), 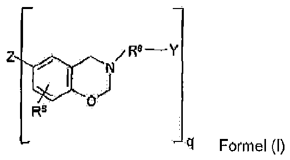 wobei q eine ganze Zahl von 1 bis 4 ist, Z aus der Gruppe bestehend aus einer direkten Bindung (wenn q 2 ist), Wasserstoff (wenn q 1 ist), Alkyl (wenn q 1 ist), Alkylen (wenn q 2 bis 4 ist), Carbonyl (wenn q 2 ist), Sauerstoff (wenn q 2 ist), Thiol (wenn q 1 ist), Schwefel (wenn q 2 ist), Sulfoxid (wenn q 2 ist) und Sulfon (wenn q 2 ist) ausgewählt ist, Y aus der Gruppe bestehend aus einer Hydroxylgruppe und einem aus substituierten oder unsubstituierten Imidazolen ausgewählten stickstoffhaltigen Heterocyclus ausgewählt ist, R⁶ eine lineare oder verzweigte zweiwertige, 1 bis 15 Kohlenstoffatome umfassende Alkylengruppe ist, welche durch ein oder mehrere aus Sauerstoff, Stickstoff und Schwefel ausgewählte Heteroatome unterbrochen sein kann, und R⁵ aus Wasserstoff, Halogen, Alkyl, Alkenyl ausgewählt ist oder R⁵ ein zweiwertiger Rest ist, der einen Naphthoxazinrest aus der Benzoxazinstruktur bildet, als Härtungsmittel für härtbare Zusammensetzungen.

15. Verfahren zur Steigerung der Polymerisationsgeschwindigkeit einer härtbaren Zusammensetzung bei Temperaturen bis 250 °C, dessen Schritte Folgendes umfassen:
a) Zusetzen mindestens einer Benzoxazinverbindung gemäß der Formel (I), 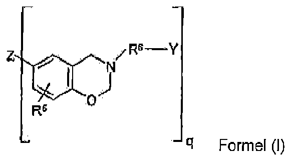 wobei q eine ganze Zahl von 1 bis 4 ist, Z aus der Gruppe bestehend aus einer direkten Bindung (wenn q 2 ist), Wasserstoff (wenn q 1 ist), Alkyl (wenn q 1 ist), Alkylen (wenn q 2 bis 4 ist), Carbonyl (wenn q 2 ist), Sauerstoff (wenn q 2 ist), Thiol (wenn q 1 ist), Schwefel (wenn q 2 ist), Sulfoxid (wenn q 2 ist) und Sulfon (wenn q 2 ist) ausgewählt ist, Y aus der Gruppe bestehend aus einer Hydroxylgruppe und einem aus substituierten oder unsubstituierten Imidazolen ausgewählten stickstoffhaltigen Heterocyclus ausgewählt ist, R⁶ eine lineare oder verzweigte zweiwertige, 1 bis 15 Kohlenstoffatome umfassende Alkylengruppe ist, welche durch ein oder mehrere aus Sauerstoff, Stickstoff und Schwefel ausgewählte Heteroatome unterbrochen sein kann, und R⁵ aus Wasserstoff, Halogen, Alkyl, Alkenyl ausgewählt ist oder R⁵ ein zweiwertiger Rest ist, der einen Naphthoxazinrest aus der Benzoxazinstruktur bildet, zu einer härtbaren Zusammensetzung;
b) Aussetzen der härtbaren Zusammensetzung Bedingungen, die für das Härten der härtbaren Zusammensetzung geeignet sind.

## Revendications

1. Composition polymérisable, comprenant
a) au moins un composé benzoxazine A selon la formule (I), 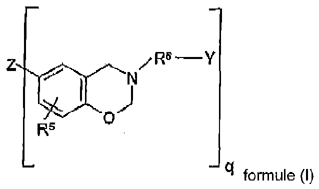 dans laquelle q est un nombre entier de 1 à 4, Z est choisi dans le groupe constitué par une liaison directe (lorsque q vaut 2), un hydrogène (lorsque q vaut 1), un alkyle (lorsque q vaut 1), un alkylène (lorsque q vaut 2 à 4), un carbonyle (lorsque q vaut 2), un oxygène (lorsque q vaut 2), un thiol (lorsque q vaut 1), un soufre (lorsque q vaut 2), un sulfoxyde (lorsque q vaut 2), et une sulfone (lorsque q vaut 2), Y est choisi dans le groupe constitué par un groupe hydroxyle et un hétérocycle contenant un atome d'azote choisi parmi les imidazoles substitués ou non substitués, R⁶ est un groupe alkylène divalent linéaire ou ramifié, comprenant 1 à 15 atomes de carbone, qui peut être interrompu par un ou plusieurs hétéroatome(s), choisi(s) parmi l'oxygène, l'azote et le soufre et R⁵ est choisi parmi l'hydrogène, un halogène, un alkyle, un alcényle ou R⁵ est un résidu divalent créant un résidu naphtoxazine hors de la structure benzoxazine, et
b) au moins un composé benzoxazine B, qui ne correspond pas à la formule (Ia), 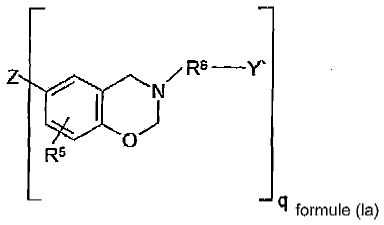 dans laquelle q, Z, R⁵ et R⁶ sont définis comme ci-dessus et Y' est choisi dans le groupe constitué par un groupe hydroxyle et un hétérocycle contenant un atome d'azote.

2. Composition polymérisable selon la revendication 1, dans laquelle R⁶ est un groupe alkylène divalent linéaire comprenant 1 à 4 atomes de carbone.

3. Composition polymérisable selon la revendication 1, dans laquelle R⁶ est un groupe alkylène divalent linéaire, qui est interrompu par au moins un atome d'oxygène.

4. Composition polymérisable selon l'une quelconque des revendications 1 à 3, dans laquelle Y est un groupe hydroxyle.

5. Composition polymérisable selon l'une quelconque des revendications 1 à 4, dans laquelle le composé benzoxazine B comprend au moins un composé benzoxazine selon la formule (II) ou la formule (IIa), 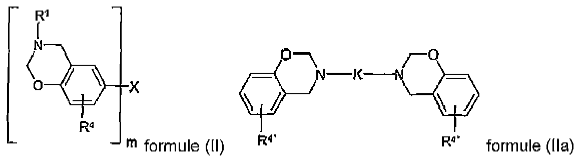 dans laquelle m est un nombre entier de 1 à 4, X est choisi dans le groupe constitué par une liaison directe (lorsque m vaut 2), un hydrogène (lorsque m vaut 1), un alkyle (lorsque m vaut 1), un alkylène (lorsque q vaut 2 à 4), un carbonyle (lorsque q vaut 2), un oxygène (lorsque q vaut 2), un thiol (lorsque q vaut 1), un soufre (lorsque m vaut 2), un sulfoxyde (lorsque m vaut 2), et une sulfone (lorsque m vaut 2), R¹ est choisi parmi l'hydrogène, un alkyle, un alcényle et un aryle et R¹ ne comprend aucun groupe hydroxyle ou tout autre hétérocycle contenant un atome d'azote, K est choisi dans le groupe constitué par le biphényle, le diphénylméthane, le diphénylisopropane, le sulfure de diphényle, le diphénylsulfoxyde, la diphénylsulfone, et la diphénylcétone, et R⁴ est choisi parmi l'hydrogène, un halogène, un alkyle, un alcényle ou R⁴ est un résidu divalent créant un résidu naphtoxazine hors de la structure benzoxazine et R⁴' est défini comme R⁴_{.}

6. Composition polymérisable selon l'une quelconque des revendications 1 à 5, dans laquelle ladite composition polymérisable comprend au moins un composé benzoxazine A et au moins un composé benzoxazine B dans un rapport molaire de 99,9:0,1: à 0,1:99,9, de préférence de 50:50 à 1:99.

7. Composition polymérisable selon l'une quelconque des revendications 1 à 6, dans laquelle la quantité totale de tous les composés benzoxazine dans la composition polymérisable est dans la gamme d'environ 10 à environ 100 pour cent en poids, sur la base de la quantité totale de la composition polymérisable.

8. Composition polymérisable selon l'une quelconque des revendications 1 à 7, dans laquelle ladite composition polymérisable comprend au moins un composant époxy.

9. Composition polymérisable selon l'une quelconque des revendications 1 à 8, dans laquelle ladite composition polymérisable est durcie à des températures de 20°C à 250°C, de préférence de 50°C à 200°C, et plus préférablement de 120°C à 180°C.

10. Produit de réaction durci de la Composition polymérisable selon l'une quelconque des revendications 1 à 9.

11. Produit de réaction durci selon la revendication 10, comprenant une couche ou un faisceau de fibres imprégné(e) de la composition polymérisable selon l'une quelconque des revendications 1 à 9 avant durcissement.

12. Procédé de production du produit de réaction durci selon la revendication 11, dont les étapes comprennent :
a) la fourniture d'une couche ou d'un faisceau de fibres;
b) la fourniture de la composition polymérisable selon l'une quelconque des revendications 1 à 9 ;
c) l'union de la composition polymérisable et de la couche ou du faisceau de fibres pour former un préimprégné ou un assemblage d'étoupe préimprégnée ; et
d) l'élimination, éventuellement, de l'excès de composition polymérisable du préimprégné ou de l'assemblage d'étoupe préimprégnée, et
l'exposition du préimprégné ou de l'assemblage d'étoupe préimprégnée résultant à une température élevée et des conditions de pression suffisantes pour imprégner la couche ou le faisceau de fibres avec la composition polymérisable pour former le produit de réaction durci.

13. Composition adhésive, d'étanchéité ou de revêtement, comprenant la composition polymérisable selon l'une quelconque des revendications 1 à 9.

14. Utilisation du au moins un composé benzoxazine selon la formule (I), 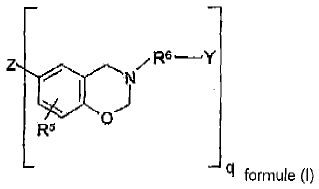 dans laquelle q est un nombre entier de 1 à 4, Z est choisi dans le groupe constitué par une liaison directe (lorsque q vaut 2), un hydrogène (lorsque q vaut 1), un alkyle (lorsque q vaut 1), un alkylène (lorsque q vaut 2 à 4), un carbonyle (lorsque q vaut 2), un oxygène (lorsque q vaut 2), un thiol (lorsque q vaut 1), un soufre (lorsque q vaut 2), un sulfoxyde (lorsque q vaut 2), et une sulfone (lorsque q vaut 2), Y est choisi dans le groupe constitué par un groupe hydroxyle et un hétérocycle contenant un atome d'azote choisi parmi les imidazoles substitués ou non substitués, R⁶ est un groupe alkylène divalent linéaire ou ramifié, comprenant 1 à 15 atomes de carbone, qui peut être interrompu par un ou plusieurs hétéroatome(s), choisi(s) parmi l'oxygène, l'azote et le soufre et R⁵ est choisi parmi l'hydrogène, un halogène, un alkyle, un alcényle ou R⁵ est un résidu divalent créant un résidu naphtoxazine hors de la structure benzoxazine, comme agent durcissant pour les compositions durcissables.

15. Procédé d'augmentation de la vitesse de polymérisation d'une composition durcissable à des températures jusqu'à 250°C, dont les étapes comprennent :
a) l'ajout d'au moins un composé benzoxazine selon la formule (I), 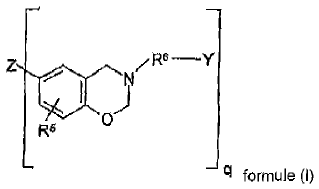 dans laquelle q est un nombre entier de 1 à 4, Z est choisi dans le groupe constitué par une liaison directe (lorsque q vaut 2), un hydrogène (lorsque q vaut 1), un alkyle (lorsque q vaut 1), un alkylène (lorsque q vaut 2 à 4), un carbonyle (lorsque q vaut 2), un oxygène (lorsque q vaut 2), un thiol (lorsque q vaut 1), un soufre (lorsque q vaut 2), un sulfoxyde (lorsque q vaut 2), et une sulfone (lorsque q vaut 2), Y est choisi dans le groupe constitué par un groupe hydroxyle et un hétérocycle contenant un atome d'azote choisi parmi les imidazoles substitués ou non substitués, R⁶ est un groupe alkylène divalent linéaire ou ramifié, comprenant 1 à 15 atomes de carbone, qui peut être interrompu par un ou plusieurs hétéroatome(s), choisi(s) parmi l'oxygène, l'azote et le soufre et R⁵ est choisi parmi l'hydrogène, un halogène, un alkyle, un alcényle ou R⁵ est un résidu divalent créant un résidu naphtoxazine hors de la structure benzoxazine ; à une composition durcissable ;
b) la soumission de la composition durcissable à des conditions appropriées pour durcir la composition durcissable.
